# EUROPEAN PATENT SPECIFICATION

(11) **EP 3 976 590 B1**
(45) Date of publication and mention of the grant of the patent: **12.04.2023**
(21) Application number: 20804378.6
(22) Date of filing: 16.10.2020
(51) Int. Cl.: C07D 231/16, C07D 401/04, C07D 405/04

(54) **METHODS FOR THE PREPARATION OF 5-BROMO-2-(3-CHLORO-PYRIDIN-2-YL)-2H-PYRAZOLE-3-CARBOXYLIC ACID**
VERFAHREN ZUR HERSTELLUNG VON 5-BROM-2-(3-CHLOR-PYRIDIN-2-YL)-2H-PYRAZOL-3-CARBONSÄURE
PROCÉDÉS DE PRÉPARATION D'ACIDE 5-BROMO-2-(3-CHLORO-PYRIDIN-2-YL)-2H-PYRAZOLE-3-CARBOXYLIQUE

(30) Priority: 18.10.2019 US 201962916832 P; 06.11.2019 US 201962931310 P
(43) Date of publication of application: 06.04.2022
(73) Proprietor: FMC CORPORATION, Philadelphia, PA 19104 (US); FMC Agro Singapore Pte. Ltd., Singapore 018983 (SG)
(72) Inventor: CAO, Yanchun, Philadelphia, PA 19104 (US); CHEN, Liang, Philadelphia, PA 19104 (US); MAO, Jianhua, Philadelphia, PA 19104 (US); XU, Zhijian, Philadelphia, PA 19104 (US)
(74) Representative: Dehns
(86) International application number: PCT/US2020/055883
(87) International publication number: WO 2021/076831

(56) References cited:
- EP-A1- 2 145 885
- WO-A1-2006/068669
- WO-A1-2013/076092
- WO-A2-2009/010260

## Description

### FIELD OF INVENTION

This disclosure is directed to novel methods of synthesizing compounds of Formula VI including 5-bromo-2-(3-chloro-pyridin-2-yl)-2H-pyrazole-3-carboxylic acid. Compounds prepared by the methods disclosed herein are useful for preparation of certain anthranilamide compounds that are of interest as insecticides, such as, for example, the insecticides chlorantraniliprole and cyantraniliprole.

### BACKGROUND

Conventional processes for the production of 5-Bromo-2-(3-chloro-pyridin-2-yl)-2H-pyrazole-3-carboxylic acid are subject to several industrial concerns, such as processability, environmental hazards, high cost, reagent reactivity, and necessary specialized equipment.

The present disclosure provides novel methods useful for preparing 5-Bromo-2-(3-chloro-pyridin-2-yl)-2H-pyrazole-3-carboxylic acid and derivatives thereof. The benefits of the methods of the present disclosure compared to previous methods are numerous and include improved overall yield, reduced cost, eliminated need for mixed solvent separations, reduced waste, simplified operation complexity, and reduced process hazards.

The disclosed methods provide an overall yield of about 50% with commercially available and easily handled reagents.

WO2006/068669A1 discloses compositions comprising 3-bromo-N-[4-cyano-2-methyl-6-[(methylamino)carbonyl]phenyl]-1-(3-chloro-2-pyridinyl)-1H-pyrazole-5-carboxamide and methods for producing this compound.

### BRIEF DESCRIPTION

In a first aspect, provided herein is a method of preparing a compound of Formula VI, wherein 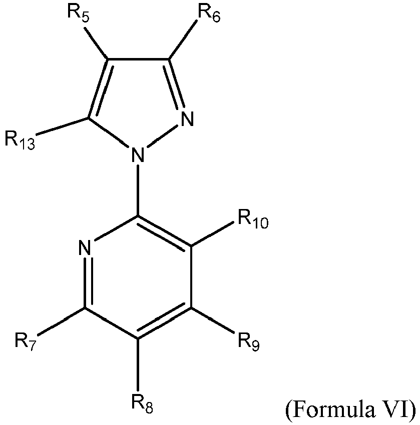 each of R₅ - R₁₀ is independently selected from hydrogen and halogen; and
R₁₃ is an organic acid, the method comprising
I) forming a mixture comprising
   A) a compound of Formula V, wherein 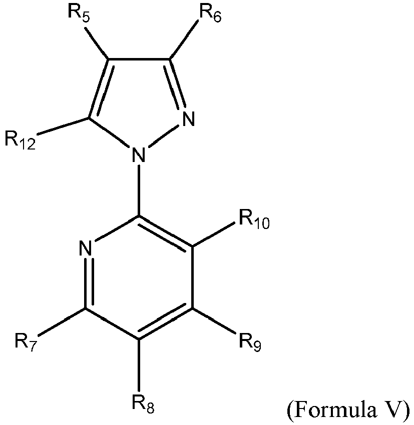
      each of R₅ - R₁₀ is independently selected from hydrogen and halogen;
      R₁₂ is selected from ester, and nitrile; and
      wherein the compound of Formula V is prepared according to a method comprising
         i) forming a mixture comprising
            a) a compound of Formula III, wherein 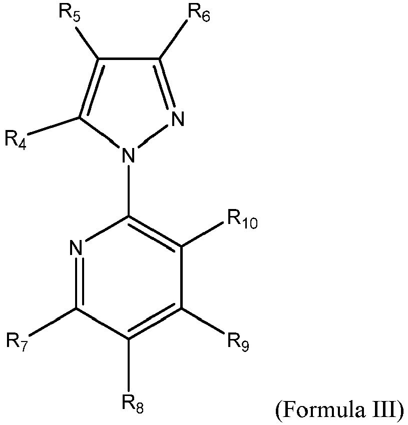
               R₄ is hydrogen;
               each of R₅ - R₁₀ is independently selected from hydrogen and halogen; and
               wherein the compound of Formula III is prepared by mixing a compound of Formula II with a compound of Formula IV in a solvent in the presence of an inorganic base and optionally an additive,
                  wherein the compound of Formula II has the structure, 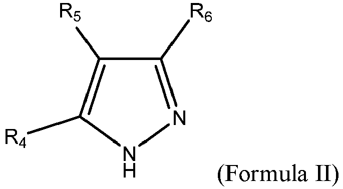 wherein each of R₄, R₅, and R₆ is independently selected from hydrogen and halogen; and
                  wherein at least one of R₄, R₅, and R₆ is hydrogen; and
                  wherein the compound of Formula IV has the structure, 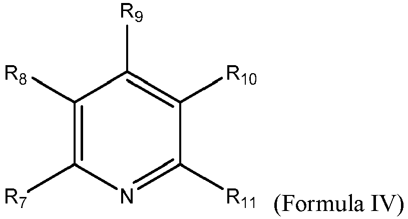 wherein each of R₇ - R₁₁ is independently selected from hydrogen and halogen; and
                     wherein the compound of Formula II is prepared according to a method comprising
                     ia) forming a mixture comprising
                        aa) a compound of Formula VIII, wherein 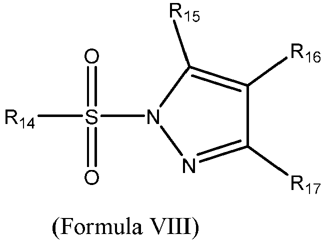
                           R₁₄ is selected from substituted or unsubstituted aryl, substituted or unsubstituted heteroaryl, substituted or unsubstituted carbocycle, and substituted or unsubstituted heterocycle;
                           each of R₁₅, R₁₆, and R₁₇ is independently selected from hydrogen and halogen; and
                           wherein at least one of R₁₅, R₁₆, and R₁₇ is a halogen;
                        bb) a solvent; and
                        cc) an inorganic base; and
                     iia) reacting the mixture;
            b) a solvent;
            c) an organic compound;
            d) a compound comprising a metal; and
            e) optionally an additive; and
         ii) reacting the mixture; and
   B) a metal hydroxide; and
II) reacting the mixture.

In a second aspect, provided herein is a method of preparing a compound of Formula VI, wherein 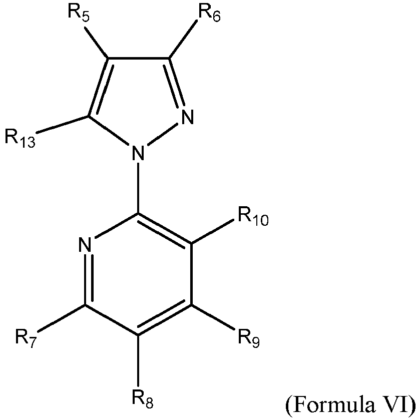 each of R₅ - R₁₀ is independently selected from hydrogen and halogen; and
R₁₃ is an organic acid, the method comprising
I) forming a mixture comprising
   A) a compound of Formula III, wherein 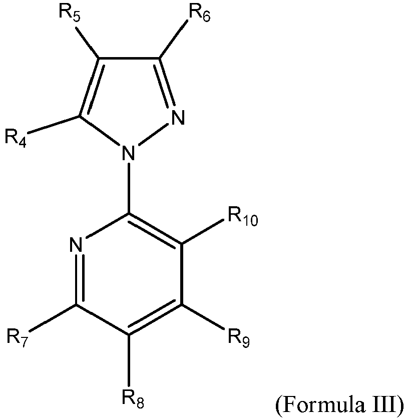
      R₄ is hydrogen;
      each of R₅ - R₁₀ is independently selected from hydrogen and halogen; and
         wherein the compound of Formula III is prepared by mixing a compound of Formula II with a compound of Formula IV in a solvent in the presence of an inorganic base and optionally an additive,
         wherein the compound of Formula II has the structure 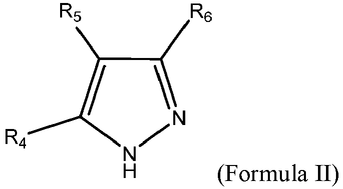 wherein each of R₄, R₅, and R₆ is independently selected from hydrogen and halogen; and
         wherein at least one of R₄, R₅, and R₆ is hydrogen; and
         wherein the compound of Formula IV has the structure 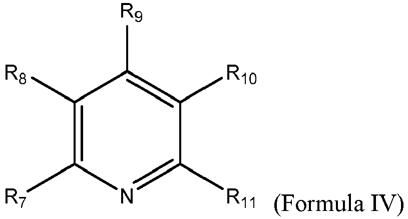 wherein each of R₇ - R₁₁ is independently selected from hydrogen and halogen; and
         wherein the compound of Formula II is prepared according to a method comprising
            IA) forming a mixture comprising
               AA) a compound of Formula VIII, wherein 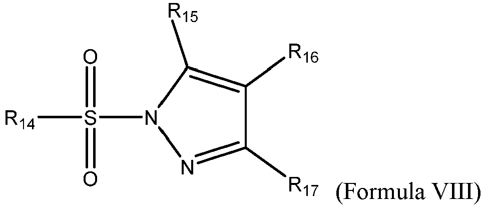
                  R₁₄ is selected from substituted or unsubstituted aryl, substituted or unsubstituted heteroaryl, substituted or unsubstituted carbocycle, and substituted or unsubstituted heterocycle;
                  each of R₁₅, R₁₆, and R₁₇ is independently selected from hydrogen and halogen; and
                  wherein at least one of R₁₅, R₁₆, and R₁₇ is a halogen;
               BB) a solvent; and
               CC) an inorganic base; and
            IIa) reacting the mixture;
   B) a carbonyl-containing compound;
   C) a solvent;
   D) a compound comprising a metal; and
   E) optionally an additive; and
II) reacting the mixture.

In the first and second aspects, the compound of Formula III may be prepared according to a method comprising
IA) forming a mixture comprising
   AA) the compound of Formula II;
   BB) the compound of Formula IV;
   CC) the solvent;
   DD) the inorganic base; and
   EE) optionally the additive; and
IIA) reacting the mixture.

In one aspect, provided herein is a method of preparing a compound of Formula II, wherein 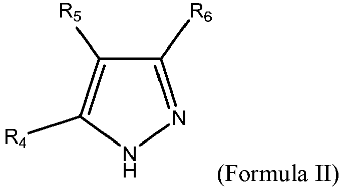 each of R₄, R₅, and R₆ is independently selected from hydrogen and halogen; and wherein at least one of R₄, R₅, and R₆ is hydrogen, the method comprising
I) forming a mixture comprising
   A) a compound of Formula VIII, wherein 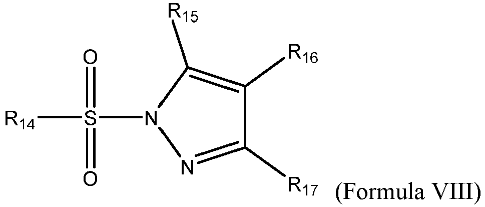
      R₁₄ is selected from substituted or unsubstituted aryl, substituted or unsubstituted heteroaryl, substituted or unsubstituted carbocycle, and substituted or unsubstituted heterocycle;
      each of R₁₅, R₁₆, and R₁₇ is independently selected from hydrogen and halogen; and
      wherein at least one of R₁₅, R₁₆, and R₁₇ is a halogen;
   B) a solvent; and
   C) an inorganic base; and
II) reacting the mixture.

As illustrated, for example, in Scheme 10 below the compound of Formula VIII can be prepared from a compound of Formula VII. A compound of Formula VII, wherein 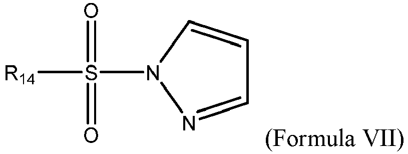 R₁₄ is selected from substituted or unsubstituted aryl, substituted or unsubstituted heteroaryl, substituted or unsubstituted carbocycle, and substituted or unsubstituted heterocycle, can be prepared by a method comprising
I) forming a mixture comprising
   A) pyrazole or a pyrazole derivative;
   B) an organosulfur compound;
   C) optionally a solvent; and
   D) optionally a base; and
II) reacting the mixture.

The compound of Formula VIII, wherein 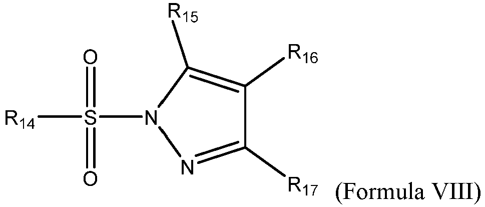
R₁₄ is selected from substituted or unsubstituted aryl, substituted or unsubstituted heteroaryl, substituted or unsubstituted carbocycle, and substituted or unsubstituted heterocycle;
each of R₁₅, R₁₆, and R₁₇ is independently selected from hydrogen and halogen; and
wherein at least one of R₁₅, R₁₆, and R₁₇ is a halogen, can be prepared by a method comprising
   I) forming a mixture comprising
      A) the compound of Formula VII, wherein 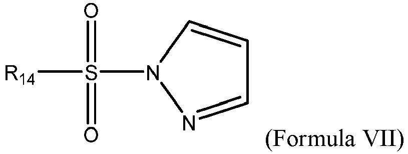 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
II) reacting the mixture.

A compound of Formula V, wherein 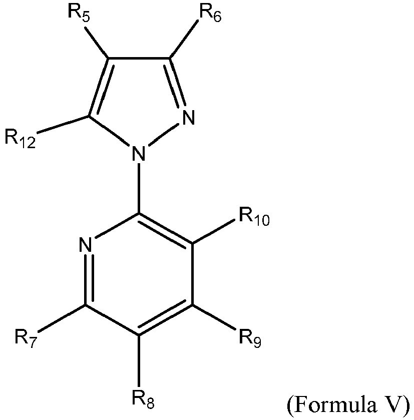 each of R₅ - R₁₀ is independently selected from hydrogen and halogen; and
R₁₂ is selected from ether, ester, and nitrile, may be prepared by a method comprising
i) forming a mixture comprising
   a) a compound of Formula III, wherein 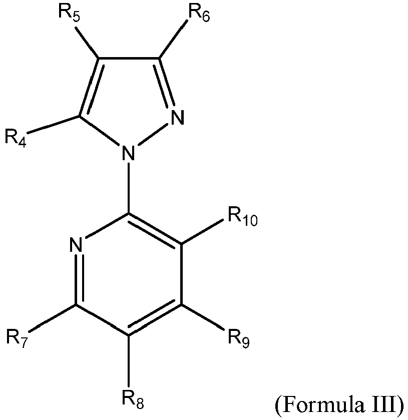
      R₄ is hydrogen;
      each of R₅ - R₁₀ is independently selected from hydrogen and halogen; and
      wherein the compound of Formula III is prepared according to a method comprising
         IA) forming a mixture comprising
            AA) a compound of Formula II, wherein 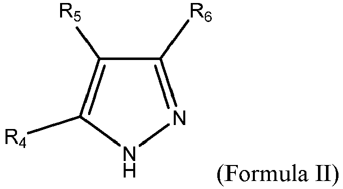
               each of R₄, R₅, and R₆ is independently selected from hydrogen and halogen;
               wherein at least one of R₄, R₅, and R₆ is hydrogen; and
               wherein the compound of Formula II is prepared according to a method comprising
                  ia) forming a mixture comprising
                     aa) a compound of Formula VIII, wherein 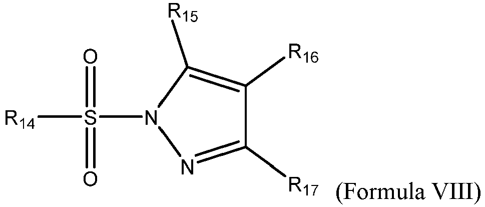
                        R₁₄ is selected from substituted or unsubstituted aryl, substituted or unsubstituted heteroaryl, substituted or unsubstituted carbocycle, and substituted or unsubstituted heterocycle;
                        each of R₁₅, R₁₆, and R₁₇ is independently selected from hydrogen and halogen; and
                        wherein at least one of R₁₅, R₁₆, and R₁₇ is a halogen;
                     bb) a solvent; and
                     cc) an inorganic base; and
                  iia) reacting the mixture;
            BB) a compound of Formula IV, wherein 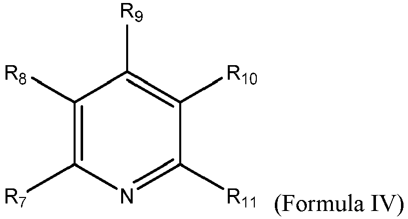 each of R₇ - R₁₁ is independently selected from hydrogen and halogen;
            CC) a solvent;
            DD) an inorganic base; and
            EE) optionally an additive; and
         IIA) reacting the mixture;
   b) a solvent;
   c) an organic compound;
   d) a compound comprising a metal; and
   e) optionally an additive; and
ii) reacting the mixture.

A compound of Formula III, wherein 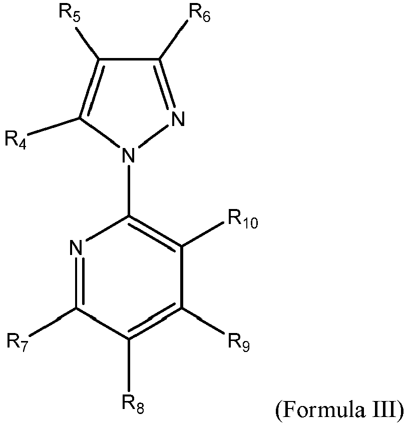 R₄ is hydrogen; and
each of R₅ - R₁₀ is independently selected from hydrogen and halogen, may be prepared by a method comprising
i) forming a mixture comprising
   a) a compound of Formula II, wherein 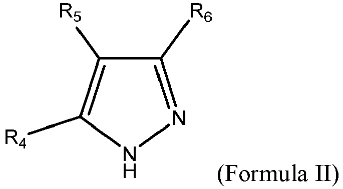
      each of R₄, R₅, and R₆ is independently selected from hydrogen and halogen;
      wherein at least one of R₄, R₅, and R₆ is hydrogen; and
      wherein the compound of Formula II is prepared according to a method comprising
         IA) forming a mixture comprising
            AA) a compound of Formula VIII, wherein 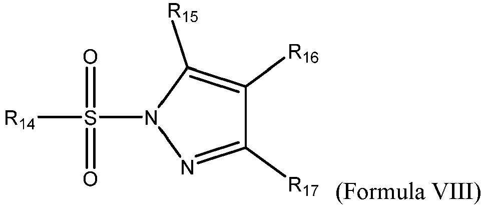
               R₁₄ is selected from substituted or unsubstituted aryl, substituted or unsubstituted heteroaryl, substituted or unsubstituted carbocycle, and substituted or unsubstituted heterocycle;
               each of R₁₅, R₁₆, and R₁₇ is independently selected from hydrogen and halogen; and
               wherein at least one of R₁₅, R₁₆, and R₁₇ is a halogen;
            BB) a solvent; and
            CC) an inorganic base; and
         IIa) reacting the mixture;
   b) a compound of Formula IV, wherein 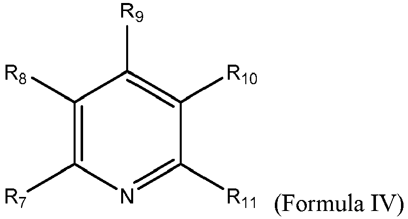 each of R₇ - R₁₁ is independently selected from hydrogen and halogen;
   c) a solvent;
   d) an inorganic base; and
   e) optionally an additive; and
ii) reacting the mixture.

A compound of Formula II-A, wherein 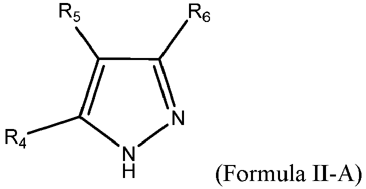
M is selected from alkali metals and alkaline metals;
each of R₄, R₅, and R₆ is independently selected from hydrogen and halogen; and
wherein at least one of R₄, R₅, and R₆ is hydrogen, can be prepared by a method comprising
   I) forming a mixture comprising
      A) a compound of Formula VIII, wherein 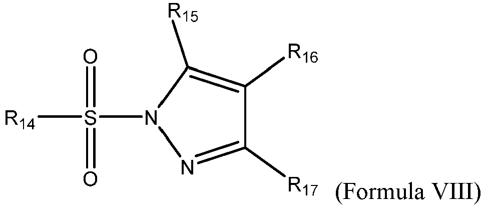
         R₁₄ is selected from substituted or unsubstituted aryl, substituted or unsubstituted heteroaryl, substituted or unsubstituted carbocycle, and substituted or unsubstituted heterocycle;
         each of R₁₅, R₁₆, and R₁₇ is independently selected from hydrogen and halogen; and
         wherein at least one of R₁₅, R₁₆, and R₁₇ is a halogen;
      B) a solvent; and
      C) an inorganic base; and
II) reacting the mixture.

Compounds of Formula II-A as disclosed herein may have the structure 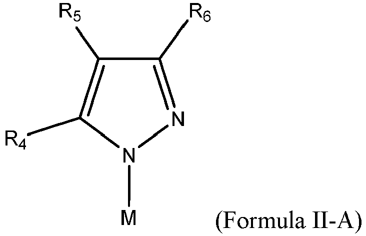 wherein:
M is selected from alkali metals and alkaline metals;
each of R₄, R₅, and R₆ is independently selected from hydrogen and halogen; and
wherein at least one of R₄, R₅, and R₆ is hydrogen.

### DETAILED DESCRIPTION OF THE DISCLOSURE

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process or method.

The transitional phrase "consisting of' excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of' is used to define a composition or method that includes materials, steps, features, components, or elements, in addition to those literally disclosed, provided that these additional materials, steps, features, components, or elements do not materially affect the basic and novel characteristic(s) of the claimed invention. The term "consisting essentially of' occupies a middle ground between "comprising" and "consisting of".

Where an invention or a portion thereof is defined with an openended term such as "comprising," it should be readily understood that (unless otherwise stated) the description should be interpreted to also describe such an invention using the terms "consisting essentially of" or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As used herein, the term "about" means plus or minus 10% of the value.

The term "halogen", either alone or in compound words such as "haloalkyl", includes fluorine, chlorine, bromine or iodine. Further, when used in compound words such as "haloalkyl", said alkyl may be partially or fully substituted with halogen atoms which may be the same or different.

When a group contains a substituent which can be hydrogen, for example R⁴, then, when this substituent is taken as hydrogen, it is recognized that this is equivalent to said group being unsubstituted.

The term "organic base" includes, without limitation, amine compounds (e.g., primary, secondary and tertiary amines), heterocycles including nitrogen-containing heterocycles, and ammonium hydroxide.

The term "inorganic base" includes, without limitation, inorganic compounds with the ability to react with, or neutralize, acids to form salts, such as, for example, metal salts of hydroxide, carbonate, bicarbonate and phosphate.

The term "halogenation reagent" includes, without limitation, halogens and inorganic compounds, such as, for example, bromine, NBS, and 1,3-dibromo-5,5-dimethylhylhydantoin.

The term "phase transfer catalyst" includes compounds that facilitate the migration of a reactant from one phase into another phase where a reaction occurs. Phase transfer catalysis refers to the acceleration of the reaction upon the addition of the phase transfer catalyst.

The term "ester" includes, without limitation, a functional group comprising an ester bond (C(=O)-O-). In some aspects, the functional group comprising an ester bond is an alkyl (or cycloalkyl) having one to eight carbon atoms, like methyl, ethyl, 1 -propyl, 2-propyl, 1 - butyl, 1-methylheptyl (meptyl), etc.

The term "ether" includes, without limitation, a functional group comprising an ether bond (C-O-C).

The term "nitrile" includes, without limitation, a functional group comprising a nitrile bond (-C≡N).

The term "carboxylic acid" includes, without limitation, a functional group comprising a carboxylic acid bond (C(=O)-OH).

The term "organic acid" includes, without limitation, a functional group that confers acidity and consists of atoms selected from carbon, nitrogen, oxygen, and hydrogen.

Certain compounds used in this invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers.

The embodiments of this disclosure include:
Embodiment 1. A method of preparing a compound of Formula VI, wherein 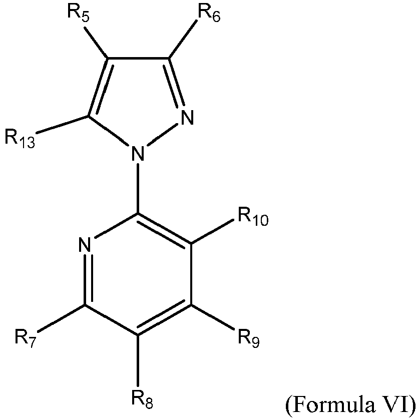 each of R₅ - R₁₀ is independently selected from hydrogen and halogen; and
   R₁₃ is an organic acid, the method comprising
   I) forming a mixture comprising
      A) a compound of Formula V, wherein 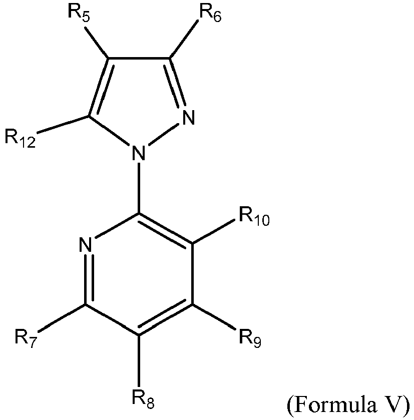
         each of R₅ - R₁₀ is independently selected from hydrogen and halogen;
         R₁₂ is selected from ester, and nitrile; and
         wherein the compound of Formula V is prepared according to a method comprising
            i) forming a mixture comprising
               a) a compound of Formula III, wherein 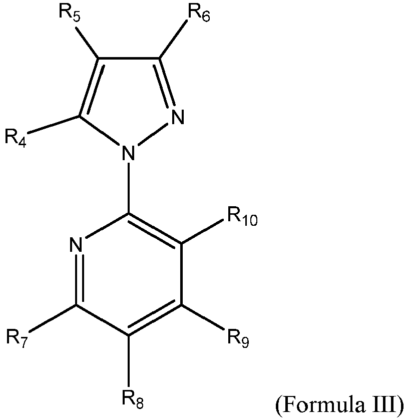
                  R₄ is hydrogen;
                  each of R₅ - R₁₀ is independently selected from hydrogen and halogen; and
                  wherein the compound of Formula III is prepared according to a method comprising
                     IA) forming a mixture comprising
                        AA) a compound of Formula II, wherein 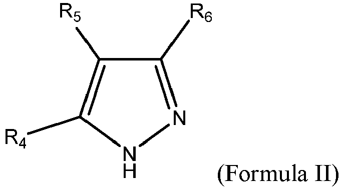
                           each of R₄, R₅, and R₆ is independently selected from hydrogen and halogen;
                           wherein at least one of R₄, R₅, and R₆ is hydrogen; and
                           wherein the compound of Formula II is prepared according to a method comprising
                              ia) forming a mixture comprising
                                 aa) a compound of Formula VIII, wherein 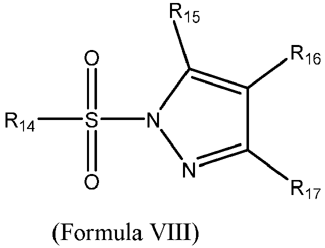
                                    R₁₄ is selected from substituted or unsubstituted aryl, substituted or unsubstituted heteroaryl, substituted or unsubstituted carbocycle, and substituted or unsubstituted heterocycle;
                                    each of R₁₅, R₁₆, and R₁₇ is independently selected from hydrogen and halogen; and
                                    wherein at least one of R₁₅, R₁₆, and R₁₇ is a halogen;
                                 bb) a solvent; and
                                 cc) an inorganic base; and
                              iia) reacting the mixture;
                        BB) a compound of Formula IV, wherein 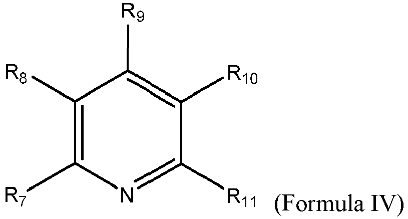 each of R₇ - R₁₁ is independently selected from hydrogen and halogen;
                        CC) a solvent;
                        DD) an inorganic base; and
                        EE) optionally an additive; and
                     IIA) reacting the mixture;
               b) a solvent;
               c) an organic compound;
               d) a compound comprising a metal; and
               e) optionally an additive; and
            ii) reacting the mixture; and
      B) a metal hydroxide; and
   II) reacting the mixture.
Embodiment 2. The method of embodiment 1, wherein the metal hydroxide is selected from alkali hydroxide, alkaline earth metal hydroxide, and combinations thereof.
Embodiment 3. The method of embodiment 2, wherein the alkali hydroxide is selected from lithium hydroxide, sodium hydroxide, and potassium hydroxide.
Embodiment 4. The method of embodiment 2, wherein the alkaline earth metal hydroxide is selected from calcium hydroxide and barium hydroxide.
Embodiment 5. The method of embodiment 1, wherein the method step II) of reacting the mixture occurs at a reaction temperature in the range of about 0 °C to about 90 °C.
Embodiment 6. The method of embodiment 1, wherein the compound comprising a metal is selected from a Grignard reagent and a lithium-containing compound.
Embodiment 7. The method of embodiment 6, wherein the Grignard reagent is selected from MeMgCl, *i*PrMgCl, *i*PrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 8. The method of embodiment 7, wherein the Grignard reagent is *i*Pr₂NMgCl.
Embodiment 9. The method of embodiment 6, wherein the lithium-containing compound is selected from LDA, nBuLi, and combinations thereof.
Embodiment 10. The method of embodiment 1, wherein the solvent b) is selected from THF, toluene, 1,4-dioxane, Me-THF, and combinations thereof.
Embodiment 11. The method of embodiment 10, wherein the solvent b) is THF.
Embodiment 12. The method of embodiment 1, wherein the organic compound is selected from dimethyl carbonate, N,N-dimethyacetamide, and combinations thereof.
Embodiment 13. The method of embodiment 12, wherein the organic compound is dimethyl carbonate.
Embodiment 14. The method of embodiment 1, wherein the method step ii) of reacting the mixture occurs at a reaction temperature in the range of about 0 °C to about 60 °C.
Embodiment 15. The method of embodiment 14, wherein the method step ii) of reacting the mixture occurs at a reaction temperature in the range of about 0 °C to about 30 °C.
Embodiment 16. The method of embodiment 1, wherein R₅ and R₆ of Formula III are each independently hydrogen.
Embodiment 17. The method of embodiment 1, wherein the inorganic base DD) is selected from powder sodium hydroxide, powder potassium hydroxide, potassium carbonate, potassium phosphate, powder sodium methoxide, powder potassium t-butoxide, and combinations thereof.
Embodiment 18. The method of embodiment 1, wherein the solvent CC) is selected from toluene, N,N-dimethylformamide, N,N-dimethylacetamide, N-methyl-2-pyrrolidone, acetonitrile, and combinations thereof.
Embodiment 19. The method of embodiment 1, wherein the additive EE) is selected from potassium iodide, a phase transfer catalyst, and combinations thereof.
Embodiment 20. The method of embodiment 19, wherein the phase transfer catalyst is selected from butyl ammonium chloride, tetra butyl ammonium bromide, aliquat-336, 18-crown-6, and combinations thereof.
Embodiment 21. The method of embodiment 1, wherein the method step IIA) of reacting the mixture occurs at a reaction temperature in the range of about 100 °C to about 200 °C.
Embodiment 22. The method of embodiment 1, wherein the solvent bb) is selected from THF, toluene, heptanes, Me-THF, and combinations thereof.
Embodiment 23. The method of embodiment 1, wherein the inorganic base cc) is selected from sodium hydroxide, potassium hydroxide, sodium carbonate, potassium carbonate, and combinations thereof.
Embodiment 24. The method of embodiment 1, wherein the method step iia) of reacting the mixture occurs at a reaction temperature in the range of about 20 °C to about 150 °C.
Embodiment 25. The method of embodiment 1, wherein the compound of Formula VIII is prepared according to a method comprising
   I) forming a mixture comprising
      A) a compound of Formula VII, wherein 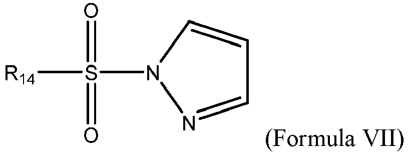 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
   II) reacting the mixture.
Embodiment 26. The method of embodiment 25, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 27. The method of embodiment 25, wherein the solvent is selected from THF, toluene, 1,4-dioxane, Me-THF, and combinations thereof.
Embodiment 28. The method of embodiment 25, wherein the compound comprising a metal is selected from a Grignard reagent and a lithium-containing compound.
Embodiment 29. The method of embodiment 28, wherein the Grignard reagent is selected from MeMgCl, *i*PrMgCl, *i*PrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 30. The method of embodiment 28, wherein the lithium-containing compound is selected from LDA, nBuLi, and combinations thereof.
Embodiment 31. The method of embodiment 30, wherein the lithium-containing compound is nBuLi.
Embodiment 32. The method of embodiment 25, wherein the method step of reacting the mixture occurs at a reaction temperature in the range of about -78 °C to about 0 °C.
Embodiment 33. The method of embodiment 32, wherein the method step of reacting the mixture occurs at a reaction temperature in the range of about -65 °C to about -50 °C.
Embodiment 34. The method of embodiment 25, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 35. The method of embodiment 34, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 36. The method of embodiment 34, wherein the solvent is selected from toluene, xylene, heptanes, and combinations thereof.
Embodiment 37. The method of embodiment 34, wherein the base is selected from pyridine, triethylamine, sodium hydroxide, sodium hydroxide solution, powder sodium acetate, and combinations thereof.
Embodiment 38. The method of embodiment 34, wherein the method step of reacting the mixture occurs at a reaction temperature in the range of about 20 °C to about 150 °C.
Embodiment 39. A method of preparing a compound of Formula VI, wherein 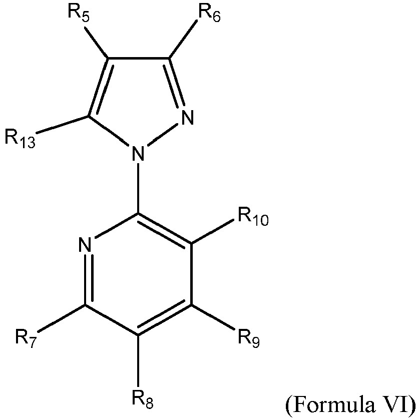 each of R₅ - R₁₀ is independently selected from hydrogen and halogen; and
   R₁₃ is an organic acid, the method comprising
   I) forming a mixture comprising
      A) a compound of Formula III, wherein 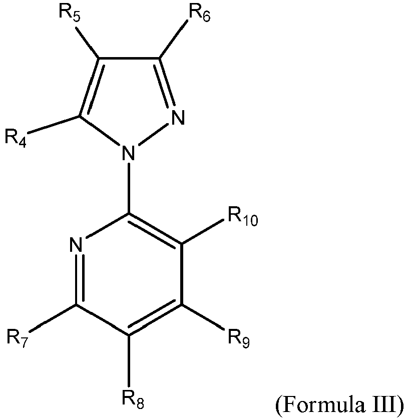
         R₄ is hydrogen;
         each of R₅ - R₁₀ is independently selected from hydrogen and halogen; and
         wherein the compound of Formula III is prepared according to a method comprising
            i) forming a mixture comprising
               a) a compound of Formula II, wherein 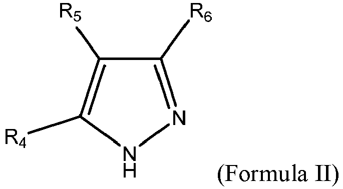
                  each of R₄, R₅, and R₆ is independently selected from hydrogen and halogen;
                  wherein at least one of R₄, R₅, and R₆ is hydrogen; and
                  wherein the compound of Formula II is prepared according to a method comprising
                     IA) forming a mixture comprising
                        AA) a compound of Formula VIII, wherein 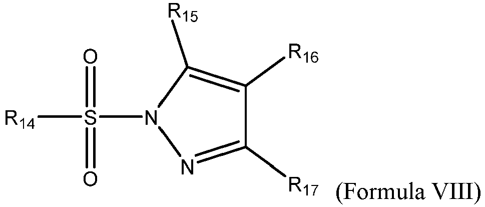
                           R₁₄ is selected from substituted or unsubstituted aryl, substituted or unsubstituted heteroaryl, substituted or unsubstituted carbocycle, and substituted or unsubstituted heterocycle;
                           each of R₁₅, R₁₆, and R₁₇ is independently selected from hydrogen and halogen; and
                           wherein at least one of R₁₅, R₁₆, and R₁₇ is a halogen;
                        BB) a solvent; and
                        CC) an inorganic base; and
                     IIa) reacting the mixture;
               b) a compound of Formula IV, wherein 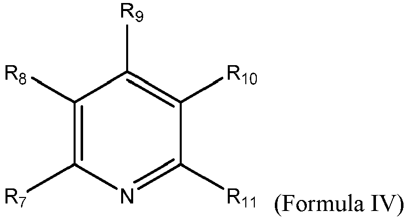 each of R₇ - R₁₁ is independently selected from hydrogen and halogen;
               c) a solvent;
               d) an inorganic base; and
               e) optionally an additive; and
            ii) reacting the mixture;
      B) a carbonyl-containing compound;
      C) a solvent;
      D) a compound comprising a metal; and
      E) optionally an additive; and
   II) reacting the mixture.
Embodiment 40. The method of embodiment 39, wherein the compound comprising a metal is selected from a Grignard reagent and a lithium-containing compound.
Embodiment 41. The method of embodiment 40, wherein the Grignard reagent is selected from MeMgCl, *i*PrMgCl, *i*PrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 42. The method of embodiment 41, wherein the Grignard reagent is *i*Pr₂NMgCl.
Embodiment 43. The method of embodiment 40, wherein the lithium-containing compound is selected from LDA, nBuLi, and combinations thereof.
Embodiment 44. The method of embodiment 39, wherein the solvent C) is selected from THF, toluene, 1,4-dioxane, Me-THF, and combinations thereof.
Embodiment 45. The method of embodiment 44, wherein the solvent C) is THF.
Embodiment 46. The method of embodiment 39, wherein the carbonyl-containing compound is selected from dimethylcarbonate, N,N-dimethylacetamide, carbon dioxide, and combinations thereof.
Embodiment 47. The method of embodiment 46, wherein the carbonyl-containing compound is carbon dioxide.
Embodiment 48. The method of embodiment 39, wherein the method step II) of reacting the mixture occurs at a reaction temperature in the range of about 0 °C to about 60 °C.
Embodiment 49. The method of embodiment 48, wherein the method step II) of reacting the mixture occurs at a reaction temperature in the range of about 0 °C to about 30 °C.
Embodiment 50. The method of embodiment 39, wherein the inorganic base d) is selected from powder sodium hydroxide, powder potassium hydroxide, potassium carbonate, potassium phosphate, powder sodium methoxide, powder potassium t-butoxide, and combinations thereof.
Embodiment 51. The method of embodiment 39, wherein the solvent c) is selected from toluene, N,N-dimethylformamide, N,N-dimethylacetamide, N-methyl-2-pyrrolidone, acetonitrile, and combinations thereof.
Embodiment 52. The method of embodiment 39, wherein the additive e) is selected from potassium iodide, a phase transfer catalyst, and combinations thereof.
Embodiment 53. The method of embodiment 52, wherein the phase transfer catalyst is selected from butyl ammonium chloride, tetra butyl ammonium bromide, aliquat-336, 18-crown-6, and combinations thereof.
Embodiment 54. The method of embodiment 39, wherein the method step ii) of reacting the mixture occurs at a reaction temperature in the range of about 100 °C to about 200 °C.
Embodiment 55. The method of embodiment 39, wherein the solvent BB) is selected from THF, toluene, heptanes, Me-THF, and combinations thereof.
Embodiment 56. The method of embodiment 39, wherein the inorganic base CC) is selected from sodium hydroxide, potassium hydroxide, sodium carbonate, potassium carbonate, and combinations thereof.
Embodiment 57. The method of embodiment 39, wherein the method step IIa) of reacting the mixture occurs at a reaction temperature in the range of about 20 °C to about 150 °C.
Embodiment 58. The method of embodiment 39, wherein the compound of Formula VIII is prepared according to a method comprising
   I) forming a mixture comprising
      A) a compound of Formula VII, wherein 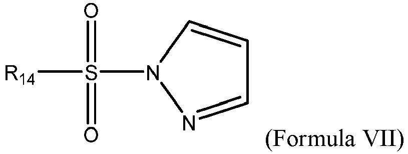 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
   II) reacting the mixture.
Embodiment 59. The method of embodiment 58, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 60. The method of embodiment 58, wherein the solvent is selected from THF, toluene, 1,4-dioxane, Me-THF, and combinations thereof.
Embodiment 61. The method of embodiment 58, wherein the compound comprising a metal is selected from a Grignard reagent and a lithium-containing compound.
Embodiment 62. The method of embodiment 61, wherein the Grignard reagent is selected from MeMgCl, *i*PrMgCl, *i*PrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 63. The method of embodiment 61, wherein the lithium-containing compound is selected from LDA, nBuLi, and combinations thereof.
Embodiment 64. The method of embodiment 63, wherein the lithium-containing compound is nBuLi.
Embodiment 65. The method of embodiment 58, wherein the method step of reacting the mixture occurs at a reaction temperature in the range of about -78 °C to about 0 °C.
Embodiment 66. The method of embodiment 65, wherein the method step of reacting the mixture occurs at a reaction temperature in the range of about -65 °C to about -50 °C.
Embodiment 67. The method of embodiment 58, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 68. The method of embodiment 67, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 69. The method of embodiment 67, wherein the solvent is selected from toluene, xylene, heptanes, and combinations thereof.
Embodiment 70. The method of embodiment 67, wherein the base is selected from pyridine, triethylamine, sodium hydroxide, sodium hydroxide solution, powder sodium acetate, and combinations thereof.
Embodiment 71. The method of embodiment 67, wherein the method step of reacting the mixture occurs at a reaction temperature in the range of about 20 °C to about 150 °C.
Embodiment 72. A method of preparing a compound of Formula II, wherein 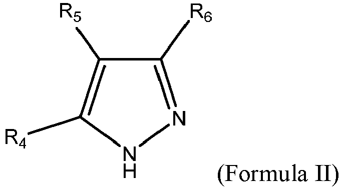 each of R₄, R₅, and R₆ is independently selected from hydrogen and halogen; and
   wherein at least one of R₄, R₅, and R₆ is hydrogen, the method comprising
   I) forming a mixture comprising
      A) a compound of Formula VIII, wherein 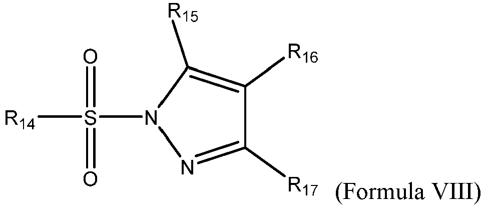
         R₁₄ is selected from substituted or unsubstituted aryl, substituted or unsubstituted heteroaryl, substituted or unsubstituted carbocycle, and substituted or unsubstituted heterocycle;
         each of R₁₅, R₁₆, and R₁₇ is independently selected from hydrogen and halogen; and
         wherein at least one of R₁₅, R₁₆, and R₁₇ is a halogen;
      B) a solvent; and
      C) an inorganic base; and
   II) reacting the mixture.
Embodiment 73. The method of embodiment 72, wherein the solvent is selected from THF, toluene, heptanes, Me-THF, and combinations thereof.
Embodiment 74. The method of embodiment 72, wherein the inorganic base is selected from sodium hydroxide, potassium hydroxide, sodium carbonate, potassium carbonate, and combinations thereof.
Embodiment 75. The method of embodiment 72, wherein the method step of reacting the mixture occurs at a reaction temperature in the range of about 20 °C to about 150 °C.
Embodiment 76. The method of embodiment 72, wherein the compound of Formula VIII is prepared according to a method comprising
   I) forming a mixture comprising
      A) a compound of Formula VII, wherein 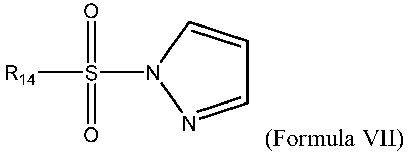 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
   II) reacting the mixture.
Embodiment 77. The method of embodiment 76, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 78. The method of embodiment 76, wherein the solvent is selected from THF, toluene, 1,4-dioxane, Me-THF, and combinations thereof.
Embodiment 79. The method of embodiment 76, wherein the compound comprising a metal is selected from a Grignard reagent and a lithium-containing compound.
Embodiment 80. The method of embodiment 79, wherein the Grignard reagent is selected from MeMgCl, *i*PrMgCl, *i*PrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 81. The method of embodiment 79, wherein the lithium-containing compound is selected from LDA, nBuLi, and combinations thereof.
Embodiment 82. The method of embodiment 81, wherein the lithium-containing compound is nBuLi.
Embodiment 83. The method of embodiment 76, wherein the method step of reacting the mixture occurs at a reaction temperature in the range of about -78 °C to about 0 °C.
Embodiment 84. The method of embodiment 83, wherein the method step of reacting the mixture occurs at a reaction temperature in the range of about -65 °C to about -50 °C.
Embodiment 85. The method of embodiment 76, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 86. The method of embodiment 85, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 87. The method of embodiment 85, wherein the solvent is selected from toluene, xylene, heptanes, and combinations thereof.
Embodiment 88. The method of embodiment 85, wherein the base is selected from pyridine, triethylamine, sodium hydroxide, sodium hydroxide solution, powder sodium acetate, and combinations thereof.
Embodiment 89. The method of embodiment 85, wherein the method step of reacting the mixture occurs at a reaction temperature in the range of about 20 °C to about 150 °C.
The compound of Formula VIII used in the methods of the invention may be prepared according to a method as defined in Embodiments 95-108.
Embodiment 95. A method of preparing a compound of Formula VIII, wherein 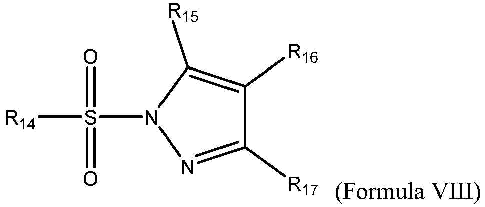
   R₁₄ is selected from substituted or unsubstituted aryl, substituted or unsubstituted heteroaryl, substituted or unsubstituted carbocycle, and substituted or unsubstituted heterocycle;
   each of R₁₅, R₁₆, and R₁₇ is independently selected from hydrogen and halogen; and
   wherein at least one of R₁₅, R₁₆, and R₁₇ is a halogen, the method comprising
      I) forming a mixture comprising
         A) the compound of Formula VII, wherein 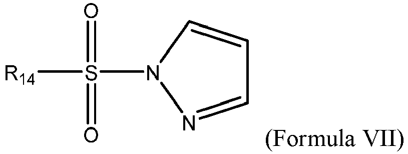 R₁₄ is selected from substituted or unsubstituted aryl, substituted or unsubstituted heteroaryl, substituted or unsubstituted carbocycle, and substituted or unsubstituted heterocycle;
         B) a halogenation reagent;
         C) a solvent; and
         D) a compound comprising a metal; and
      II) reacting the mixture.
Embodiment 96. The method of embodiment 95, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 97. The method of embodiment 90, wherein the solvent is selected from toluene, xylene, heptanes, and combinations thereof.
Embodiment 98. The method of embodiment 95, wherein the compound comprising a metal is selected from a Grignard reagent and a lithium-containing compound.
Embodiment 99. The method of embodiment 98, wherein the Grignard reagent is selected from MeMgCl, *i*PrMgCl, *i*PrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 100. The method of embodiment 98, wherein the lithium-containing compound is selected from LDA, nBuLi, and combinations thereof.
Embodiment 101. The method of embodiment 100, wherein the lithium-containing compound is nBuLi.
Embodiment 102. The method of embodiment 95, wherein the method step of reacting the mixture occurs at a reaction temperature in the range of about -78 °C to about 0 °C.
Embodiment 103. The method of embodiment 102, wherein the method step of reacting the mixture occurs at a reaction temperature in the range of about -65 °C to about -50 °C.
Embodiment 104. The method of embodiment 95, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 105. The method of embodiment 104, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 106. The method of embodiment 104, wherein the solvent is selected from toluene, xylene, heptanes, and combinations thereof.
Embodiment 107. The method of embodiment 104, wherein the base is selected from pyridine, triethylamine, sodium hydroxide, sodium hydroxide solution, powder sodium acetate, and combinations thereof.
Embodiment 108. The method of embodiment 104, wherein the method step of reacting the mixture occurs at a reaction temperature in the range of about 20 °C to about 150 °C.
The compound of Formula (III) used in the methods of the invention may be prepared according to a method as defined in Embodiments 143-166.
Embodiment 143. A method of preparing a compound of Formula III, wherein 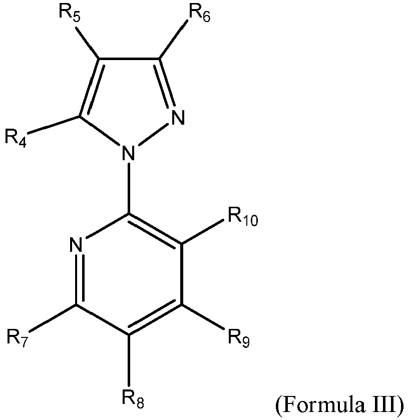 R₄ is hydrogen; and
   each of R₅ - R₁₀ is independently selected from hydrogen and halogen, the method comprising
   i) forming a mixture comprising
      a) a compound of Formula II, wherein 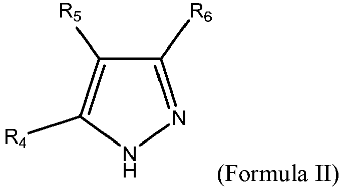
         each of R₄, R₅, and R₆ is independently selected from hydrogen and halogen;
         wherein at least one of R₄, R₅, and R₆ is hydrogen; and
         wherein the compound of Formula II is prepared according to a method comprising
            IA) forming a mixture comprising
               AA) a compound of Formula VIII, wherein 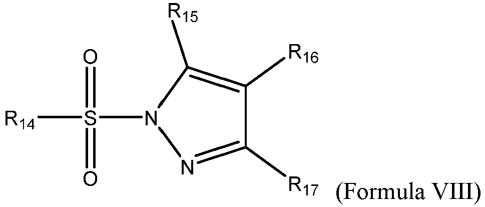
                  R₁₄ is selected from substituted or unsubstituted aryl, substituted or unsubstituted heteroaryl, substituted or unsubstituted carbocycle, and substituted or unsubstituted heterocycle;
                  each of R₁₅, R₁₆, and R₁₇ is independently selected from hydrogen and halogen; and
                  wherein at least one of R₁₅, R₁₆, and R₁₇ is a halogen;
               BB) a solvent; and
               CC) an inorganic base; and
            IIa) reacting the mixture;
      b) a compound of Formula IV, wherein 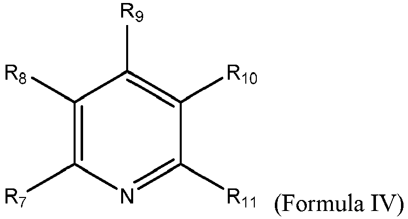 each of R₇ - R₁₁ is independently selected from hydrogen and halogen;
      c) a solvent;
      d) an inorganic base; and
      e) optionally an additive; and
   ii) reacting the mixture.
Embodiment 144. The method of embodiment 143, wherein R₅ and R₆ of Formula III are each independently hydrogen.
Embodiment 145. The method of embodiment 143, wherein the inorganic base d) is selected from powder sodium hydroxide, powder potassium hydroxide, potassium carbonate, potassium phosphate, powder sodium methoxide, powder potassium *t*-butoxide, and combinations thereof.
Embodiment 146. The method of embodiment 143, wherein the solvent c) is selected from toluene, N,N-dimethylformamide, N,N-dimethylacetamide, N-methyl-2-pyrrolidone, acetonitrile, and combinations thereof.
Embodiment 147. The method of embodiment 143, wherein the additive e) is selected from potassium iodide, a phase transfer catalyst, and combinations thereof.
Embodiment 148. The method of embodiment 147, wherein the phase transfer catalyst is selected from butyl ammonium chloride, tetra butyl ammonium bromide, aliquat-336, 18-crown-6, and combinations thereof.
Embodiment 149. The method of embodiment 143, wherein the method step ii) of reacting the mixture occurs at a reaction temperature in the range of about 100 °C to about 200 °C.
Embodiment 150. The method of embodiment 143, wherein the solvent BB) is selected from THF, toluene, heptanes, Me-THF, and combinations thereof.
Embodiment 151. The method of embodiment 143, wherein the inorganic base CC) is selected from sodium hydroxide, potassium hydroxide, sodium carbonate, potassium carbonate, and combinations thereof.
Embodiment 152. The method of embodiment 143, wherein the method step IIa) of reacting the mixture occurs at a reaction temperature in the range of about 20 °C to about 150 °C.
Embodiment 153. The method of embodiment 143, wherein the compound of Formula VIII is prepared according to a method comprising
   I) forming a mixture comprising
      A) a compound of Formula VII, wherein 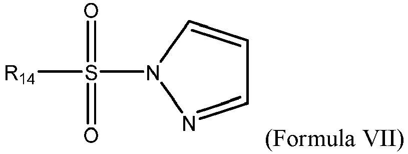 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
   II) reacting the mixture.
Embodiment 154. The method of embodiment 153, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 155. The method of embodiment 153, wherein the solvent is selected from THF, toluene, 1,4-dioxane, Me-THF, and combinations thereof.
Embodiment 156. The method of embodiment 153, wherein the compound comprising a metal is selected from a Grignard reagent and a lithium-containing compound.
Embodiment 157. The method of embodiment 156, wherein the Grignard reagent is selected from MeMgCl, *i*PrMgCl, *i*PrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 158. The method of embodiment 156, wherein the lithium-containing compound is selected from LDA, nBuLi, and combinations thereof.
Embodiment 159. The method of embodiment 158, wherein the lithium-containing compound is nBuLi.
Embodiment 160. The method of embodiment 153, wherein the method step of reacting the mixture occurs at a reaction temperature in the range of about -78 °C to about 0°C.
Embodiment 161. The method of embodiment 160, wherein the method step of reacting the mixture occurs at a reaction temperature in the range of about -65 °C to about -50 °C.
Embodiment 162. The method of embodiment 153, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 163. The method of embodiment 162, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 164. The method of embodiment 162, wherein the solvent is selected from toluene, xylene, heptanes, and combinations thereof.
Embodiment 165. The method of embodiment 162, wherein the base is selected from pyridine, triethylamine, sodium hydroxide, sodium hydroxide solution, powder sodium acetate, and combinations thereof.
Embodiment 166. The method of embodiment 162, wherein the method step of reacting the mixture occurs at a reaction temperature in the range of about 20 °C to about 150 °C.
   A compound of Formula II-A may be prepared by a method as defined in Embodiments 167-185.
Embodiment 167. A method of preparing a compound of Formula II-A, wherein 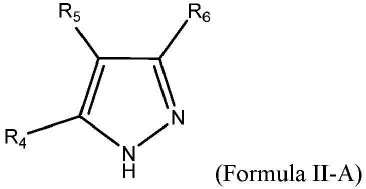
   M is selected from alkali metals and alkaline metals;
   each of R₄, R₅, and R₆ is independently selected from hydrogen and halogen; and
   wherein at least one of R₄, R₅, and R₆ is hydrogen, the method comprising
      I) forming a mixture comprising
         A) a compound of Formula VIII, wherein 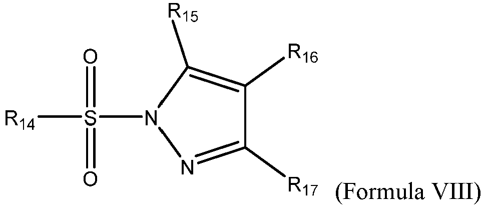
            R₁₄ is selected from substituted or unsubstituted aryl, substituted or unsubstituted heteroaryl, substituted or unsubstituted carbocycle, and substituted or unsubstituted heterocycle;
            each of R₁₅, R₁₆, and R₁₇ is independently selected from hydrogen and halogen; and
            wherein at least one of R₁₅, R₁₆, and R₁₇ is a halogen;
         B) a solvent; and
         C) an inorganic base; and
      II) reacting the mixture.
Embodiment 168. The method of embodiment 167, wherein the solvent is selected from THF, toluene, heptanes, Me-THF, and combinations thereof.
Embodiment 169. The method of embodiment 167, wherein the inorganic base is selected from sodium hydroxide, potassium hydroxide, sodium carbonate, potassium carbonate, and combinations thereof.
Embodiment 170. The method of embodiment 167, wherein the method step of reacting the mixture occurs at a reaction temperature in the range of about 20 °C to about 150 °C.
Embodiment 171. The method of claim 167, wherein M is selected from lithium, sodium, potassium, calcium, and magnesium.
Embodiment 172. The method of embodiment 167, wherein the compound of Formula VIII is prepared according to a method comprising
   I) forming a mixture comprising
      A) a compound of Formula VII, wherein 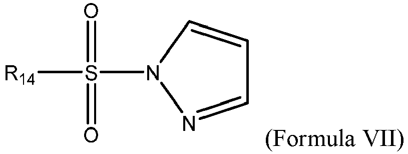 R₁₄ is selected from substituted or unsubstituted aryl, substituted or unsubstituted heteroaryl, substituted or unsubstituted carbocycle, and substituted or unsubstituted heterocycle;
      B) a halogenation reagent;
      C) a solvent; and
      D) a compound comprising a metal; and
   II) reacting the mixture.
Embodiment 173. The method of embodiment 172, wherein the halogenation reagent comprises
   A) a reagent selected from hydrogen bromide, bromine, N-bromosuccinimide, 1,3-dibromo-5,5-dimethylhylhydantoin, sodium bromide, potassium bromide, 1,2-dibromotetrachloroethane, and combinations thereof; and
   B) optionally hydrogen peroxide.
Embodiment 174. The method of embodiment 172, wherein the solvent is selected from THF, toluene, 1,4-dioxane, Me-THF, and combinations thereof.
Embodiment 175. The method of embodiment 172, wherein the compound comprising a metal is selected from a Grignard reagent and a lithium-containing compound.
Embodiment 176. The method of embodiment 172, wherein the Grignard reagent is selected from MeMgCl, *i*PrMgCl, *i*PrMgBr, EtMgCl, *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof.
Embodiment 177. The method of embodiment 172, wherein the lithium-containing compound is selected from LDA, nBuLi, and combinations thereof.
Embodiment 178. The method of embodiment 177, wherein the lithium-containing compound is nBuLi.
Embodiment 179. The method of embodiment 172, wherein the method step of reacting the mixture occurs at a reaction temperature in the range of about -78 °C to about 0°C.
Embodiment 180. The method of embodiment 179, wherein the method step of reacting the mixture occurs at a reaction temperature in the range of about -65 °C to about -50 °C.
Embodiment 181. The method of embodiment 172, wherein the compound of Formula VII is prepared according to a method comprising
   I) forming a mixture comprising
      A) pyrazole or a pyrazole derivative;
      B) an organosulfur compound;
      C) optionally a solvent; and
      D) optionally a base; and
   II) reacting the mixture.
Embodiment 182. The method of embodiment 181, wherein the organosulfur compound is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride and combinations thereof.
Embodiment 183. The method of embodiment 181, wherein the solvent is selected from toluene, xylene, heptanes, and combinations thereof.
Embodiment 184. The method of embodiment 181, wherein the base is selected from pyridine, triethylamine, sodium hydroxide, sodium hydroxide solution, powder sodium acetate, and combinations thereof.
Embodiment 185. The method of embodiment 181, wherein the method step of reacting the mixture occurs at a reaction temperature in the range of about 20 °C to about 150 °C.

Compounds of Formula II-A as disclosed herein may have the following structure 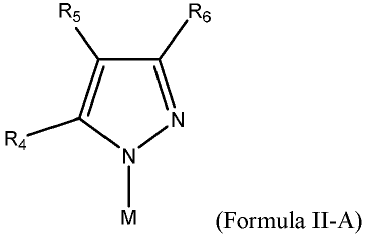 wherein
M is selected from alkali metals and alkaline metals;
each of R₄, R₅, and R₆ is independently selected from hydrogen and halogen; and
wherein at least one of R₄, R₅, and R₆ is hydrogen.

An example of a compound of Formula II-A is

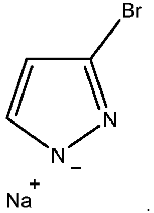

In one aspect, a compound of Formula VI is prepared according to a method represented by Scheme 1. The R groups are as defined anywhere in this disclosure.

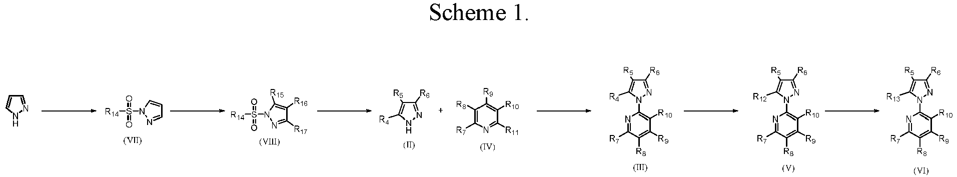

In one aspect, a compound of Formula VI is prepared according to a method represented by Scheme 2. The R groups are as defined anywhere in this disclosure.

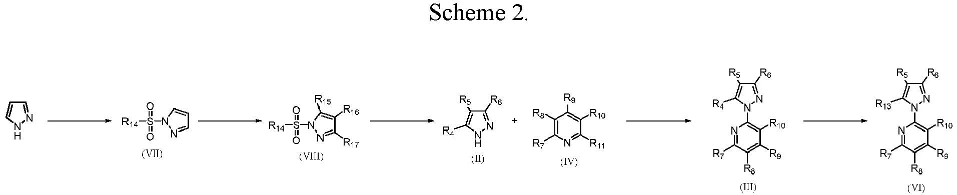

In one aspect, 5-Bromo-2-(3-chloro-pyridin-2-yl)-2H-pyrazole-3-carboxylic acid is prepared according to a method represented by Scheme 3.

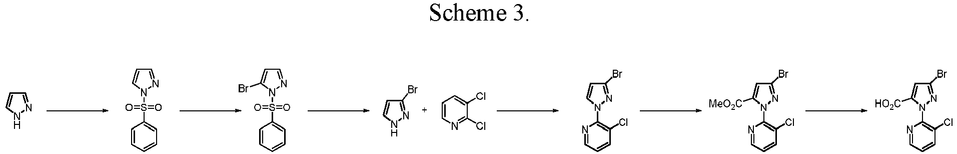

In one aspect, 5-Bromo-2-(3-chloro-pyridin-2-yl)-2H-pyrazole-3-carboxylic acid is prepared according to a method represented by Scheme 4.

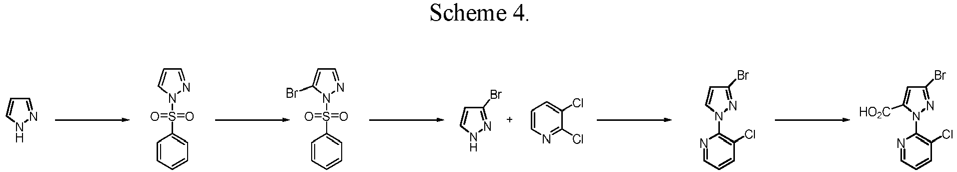

A compound of Formula VII may be prepared according to a method represented by Scheme 9. The R groups are as defined anywhere in this disclosure.

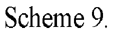

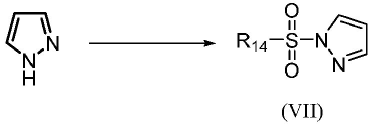

This may include reacting pyrazole with a protective group reagent optionally in the presence of a base and optionally in an organic solvent. In one embodiment, the protective group reagent is selected from benzenesulfonyl chloride, 4-methyl-benzenesulfonyl chloride, 3-methyl-benzenesulfonyl chloride, 2-methyl-benzenesulfonyl chloride, 4-ethyl-benzenesulfonyl chloride, and combinations thereof. In another embodiment, the protective reagent is benzenesulfonyl chloride. In one embodiment, the organic solvent is selected from toluene, xylene, heptanes, or combinations thereof. In another embodiment, the organic solvent is toluene. In one embodiment, the base is selected from an organic base and an inorganic base. In another embodiment, the base is selected from pyridine, triethylamine, sodium hydroxide, sodium hydroxide solution, powder sodium acetate, and combinations thereof. In another embodiment, there is no base. In one embodiment, the reaction temperature is in the range from about 20 °C to about 150 °C. In another embodiment, the reaction temperature is in the range from about 50 °C to about 90 °C.

A compound of Formula VIII may be prepared according to a method represented by Scheme 10. The R groups are as defined anywhere in this disclosure.

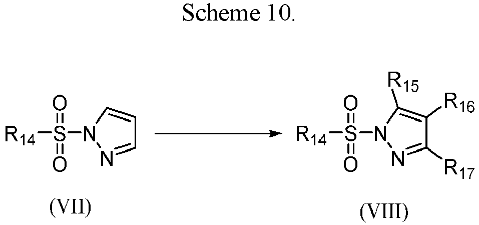

This may include reacting a compound of Formula VII with a halogenation reagent in an organic solvent in the presence of a base reagent. In one embodiment, the halogenation reagent is selected from Br₂, N-Bromosuccinimide, 1,3-Dibromo-5,5-dimethylhylhydantoin, 1,2-dibromotetrachloroethane (DBTCE), and combinations thereof. In another embodiment, the halogenation reagent is Br₂. In one embodiment, the organic solvent is selected from THF, toluene, 1,4-dioxane, Me-THF, and combinations thereof. In another embodiment, the solvent is THF. In one embodiment, base reagent is selected from LDA, nBuLi, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof. In another embodiment, the base is nBuLi. In one embodiment, the reaction temperature is in the range from about -78 °C to about 0 °C. In another embodiment, the reaction temperature is in the range from about -65 °C to about -50 °C.

In one aspect, a compound of Formula II is prepared according to a method represented by Scheme 11. The R groups are as defined anywhere in this disclosure.

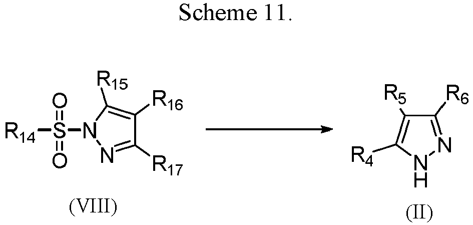

This aspect includes reacting a compound of Formula VIII with an organic solvent in the presence of an inorganic base. In one embodiment, the solvent is selected from THF, toluene, heptanes, Me-THF, and combinations thereof. In another embodiment, the solvent is toluene. In one embodiment, the inorganic base is selected from NaOH, KOH, Na₂CO₃, K₂CO₃, and combinations thereof. In another embodiment, the inorganic base is NaOH. In one embodiment, the reaction temperature is in the range from about 20 °C to about 150 °C. In another embodiment, the reaction temperature is in the range from about 50 °C to about 120 °C.

A compound of Formula II-A may be prepared according to a method represented by Scheme 12. The R groups are as defined anywhere in this disclosure.

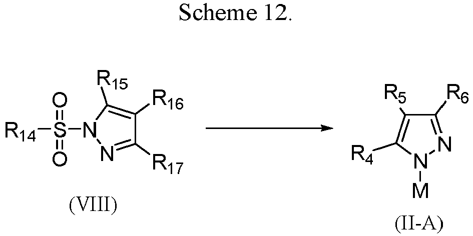

The compound of Formula II-A may be a metal salt of Formula II. The bond between M and N may be an ionic bond. M may be selected from alkali metals and alkaline metals. M may be selected from lithium, sodium, and potassium, such as sodium. M may be selected from calcium and magnesium.

One example of a compound of Formula II-A is

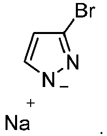

This method may include reacting a compound of Formula VIII with an organic solvent in the presence of an inorganic base. The solvent may be selected from THF, toluene, heptanes, Me-THF, and combinations thereof, such as toluene. The inorganic base may be selected from NaOH, KOH, Na₂CO₃, K₂CO₃, and combinations thereof, such as NaOH. The reaction temperature may be in the range from about 20 °C to about 150 °C, such as from about 50 °C to about 120 °C.

A compound of Formula III may be prepared according to a method represented by Scheme 13. The R groups are as defined anywhere in this disclosure.

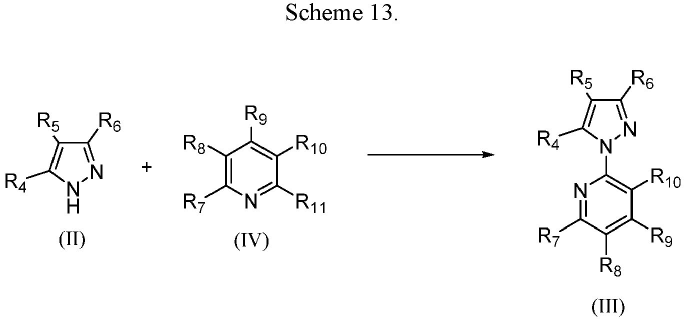

This method may include mixing a compound of Formula II with a compound of Formula IV in a solvent in the presence of an inorganic base and optionally an additive. In one embodiment, the inorganic base is selected from powder sodium hydroxide, powder potassium hydroxide, potassium carbonate, potassium phosphate powder sodium methoxide, powder potassium *t*-butoxide, and combinations thereof. In one embodiment, the solvent is selected from toluene, N,N-Dimethylformamide (DMF), N,N-Dimethylacetamide (DMAc), N-methyl-2-pyrrolidone (NMP), acetonitrile, and combinations thereof. In another embodiment, the solvent is toluene. In one embodiment, the reaction temperature is in the range from about 100 °C to about 200 °C. In another embodiment, the reaction temperature is in the range from about 130 °C to about 180 °C. In another embodiment, the temperature is in the range from about 145 °C to about 160 °C.

In one aspect, a compound of Formula VI is prepared according to a method represented by Scheme 14. The R groups are as defined anywhere in this disclosure.

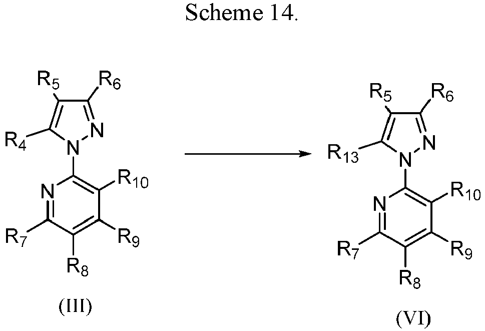

This aspect includes mixing a compound of Formula III with CO₂ in a solvent in the presence of a base reagent and optionally an additive. In one embodiment, the base reagent is selected from MeMgCl, *i*PrMgCl, *i*PrMgBr, EtMgCl, LDA, nBuLi, *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof. In another embodiment, the base reagent is *i*Pr₂NMgCl. In one embodiment, the solvent is selected from THF, toluene, 1,4-dioxane, Me-THF, and combinations thereof. In another embodiment, the solvent is THF. In one embodiment, the reaction temperature is in the range from about 0 °C to about 60 °C. In another embodiment, the temperature is in the range from about 0 °C to about 30 °C.

A compound of Formula V may be prepared according to a method represented by Scheme 15. The R groups are as defined anywhere in this disclosure.

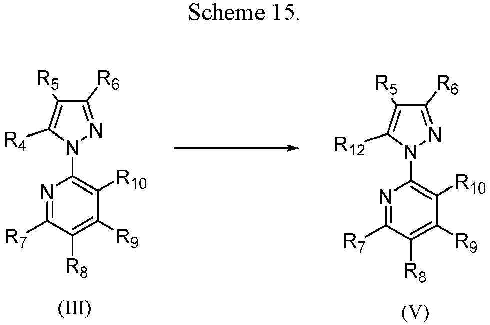

This method may include mixing a compound of Formula III with dimethyl carbonate (DMC) in a solvent in the presence of a base reagent and optionally an additive. In one embodiment, the organic base reagent is selected from MeMgCl, *i*PrMgCl, *i*PrMgBr, EtMgCl, LDA, nBuLi, *i*Pr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof. In another embodiment, the organic base is *i*Pr₂NMgCl. In one embodiment, the solvent is selected from THF, toluene, 1,4-dioxane, Me-THF, and combinations thereof. In another embodiment, the solvent is THF. In one embodiment, the reaction temperature is in the range from about 0 °C to about 60 °C. In another embodiment, the temperature is in the range from about 0 °C to about 30 °C.

In one aspect, a compound of Formula VI is prepared according to a method represented by Scheme 16. The R groups are as defined anywhere in this disclosure.

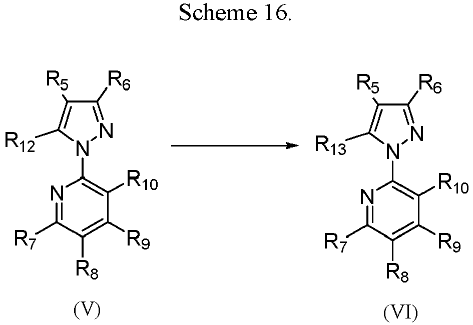

This aspect includes reacting a compound of Formula V with an aqueous metal hydroxide solution. In one embodiment, the metal hydroxide is selected from alkali hydroxide, alkaline earth metal hydroxide, and combinations thereof. In one embodiment, the alkali hydroxide is selected from lithium hydroxide, sodium hydroxide, potassium hydroxide, and combinations. In one embodiment, the alkaline earth metal hydroxide is selected from calcium hydroxide, barium hydroxide, and combinations thereof. In another embodiment, the metal hydroxide is sodium hydroxide or potassium hydroxide. In one embodiment, the reaction temperature is in the range from about 0 °C to about 90 °C. In another embodiment, the reaction temperature is in the range from about 60 °C to about 80 °C. In another embodiment, the reaction temperature is in the range from about 40 °C to about 70 °C.

### EXAMPLES

Without further elaboration, it is believed that one skilled in the art using the preceding description can utilize the present invention to its fullest extent. The following Examples are, therefore, to be construed as merely illustrative, and not limiting of the disclosure in any way whatsoever. The starting material for the following Examples may not have necessarily been prepared by a particular preparative run whose procedure is described in other Examples. It also is understood that any numerical range recited herein includes all values from the lower value to the upper value. For example, if a range is stated as 10-50, it is intended that values such as 12-30, 20-40, or 30-50, etc., are expressly enumerated in this specification. These are only examples of what is specifically intended, and all possible combinations of numerical values between and including the lowest value and the highest value enumerated are to be considered to be expressly stated in this application.

Example 1. Protecting reaction.

50 grams of pyrazole was dissolved in 150 g of toluene, and then 29.6 g of NaOH was charged and 136 g of benzenesulfonyl chloride was charged. The reaction was heated to reflux for 2 hours and then cooled to 25 °C. 100 g of 5% NaHCO₃ was charged, and stirred for 1 hour, and then the organic layer was separated and distilled to yield 147 g of Bz-protected pyrazole as product.

Example 2. Halogenation.

50 grams of Bz-protected pyrazole was dissolved in 500 g of dry THF and this mixture was cooled to -50 °C. Next, 132 ml of BuLi (2.5 M in hexane) was added dropwise at a temperature below -50 °C. After addition, the reaction was stirred at -50 °C for 1 hour and then 73.6 g of dry Br₂ was added dropwise at -50 °C. After addition, the reaction was quenched by 15% HOAc and the organic layer was collected and distilled to yield 64 g of 5-bromo-1-(phenylsulfonyl)-1H-pyrazole.

Example 3. Removal of protecting group.

5-bromo-1-(phenylsulfonyl)-1H-pyrazole was dissolved in toluene and 22 g of NaOH was charged. Next, the reaction was heated to reflux for 1 hour and then water was removed by azeotropic distillation. Then the mixture was cooled to 25 °C and used for subsequent steps.

Example 4. Coupling reaction.

11.5 grams of 3-bromo-1H-pyrazole and 14.7 g of 40% NaOH solution were reacted in the presence of toluene and at a temperature of 150 to 160 °C. Water was removed by azetropic distillation under reflux temperature to yield the corresponding 3-bromo-1H-pyrazole sodium salt. Then, toluene and 13.5 g of 2,3-dichloropyridine were added and the mixture was reacted at 150-160 °C. After reaction, the reaction mixture was quenched with water and the organic layer was separated. The organic layer was then washed with water and concentrated under vacuum at 40-45 °C to obtain 2-(3-bromo-1H-pyrazol-1-yl)-3-chloropyridine as a solid.

Example 5. Reaction in the presence of a Grignard reagent.

32 grams of 2-(3-bromo-1H-pyrazol-1-yl)-3-chloropyridine was dissolved in THF then iPr₂NMgCl (in situ MeMgCl and iPr₂NH) was added at 0 °C to yield the corresponding 2-(3-bromo-1H-pyrazol-1-yl)-3-chloropyridine magnesium salt. After 2.5 hours, 52.0 g of DMC was added drop-wise at room temperature over 6 hours. The reaction temperature was controlled at 20-40 °C. After reaction, THF and DMC were distilled off under reduced pressure, and then the reaction mixture was quenched with water. Next, toluene was added. After separation and concentration, 36.7 g of high purity (90%, LC Area) of methyl 3-bromo-1-(3-chloropyridin-2-yl)-1H-pyrazole-5-carboxylate was obtained.

Example 6. Hydrolysis.

30 grams of methyl 3-bromo-1-(3-chloropyridin-2-yl)-1H-pyrazole-5-carboxylate and a caustic soda solution in 90 g of toluene were charged to a flask at 85°C. The the mixture was kept at 80-85 °C for 2 hours to complete reaction. The aqueous phase was washed with toluene twice. Dilute H₂SO₄ was used to adjust pH to about 1. After filtration and drying, 26.5 g (97%, LC Area) of 5-bromo-2-(3-chloro-pyridin-2-yl)-2H-pyrazole-3-carboxylic acid was obtained.

Example 7. Carboxylation.

32 grams of 2-(3-bromo-1H-pyrazol-1-yl)-3-chloropyridine was dissolved in THF and then iPr₂NMgCl, obtained in situ by separately adding MeMgCl and iPr₂NH, was added at 0 °C to yield the corresponding 2-(3-bromo-1H-pyrazol-1-yl)-3-chloropyridine magnesium salt. After 2.5 hours, CO₂ gas was added at room temperature over 1 hour. The reaction temperature was controlled at 20-40 °C. After reaction, THF was distilled off under reduced pressure, and then the reaction mixture was quenched with water. Next, toluene was added. After separation and concentration, 30.0 g of high purity (95%, LC Area) of 5-bromo-2-(3-chloro-pyridin-2-yl)-2H-pyrazole-3-carboxylic acid was obtained.

This written description uses examples to illustrate the present disclosure, including the best mode, and also to enable any person skilled in the art to practice the disclosure, including making and using any devices or systems and performing any incorporated methods. The scope of the disclosure is defined by the claims, and may include other examples that occur to those skilled in the art.

## Claims

1. A method of preparing a compound of Formula VI, wherein 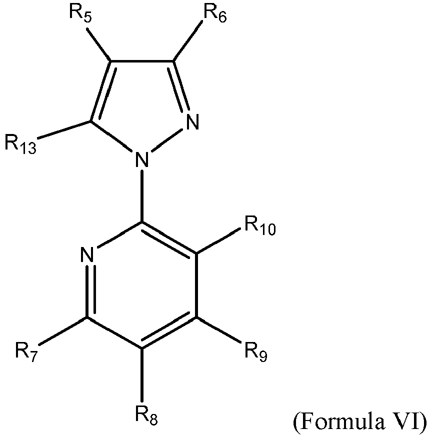 each of R₅ - R₁₀ is independently selected from hydrogen and halogen; and
R₁₃ is an organic acid, the method comprising
I) forming a mixture comprising
A) a compound of Formula V, wherein 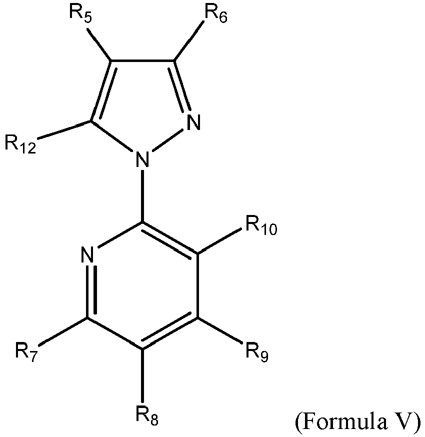
each of R₅ - R₁₀ is independently selected from hydrogen and halogen;
R₁₂ is selected from ester, and nitrile; and
wherein the compound of Formula V is prepared according to a method comprising
i) forming a mixture comprising
a) a compound of Formula III, wherein 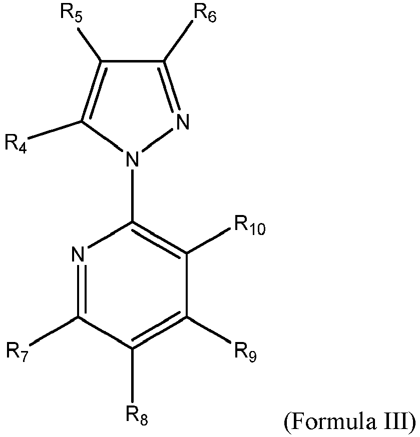 R₄ is hydrogen;
each of R₅ - R₁₀ is independently selected from hydrogen and halogen; and
wherein the compound of Formula III is prepared by mixing a compound of Formula II with a compound of Formula IV in a solvent in the presence of an inorganic base and optionally an additive,
wherein the compound of Formula II has the structure, 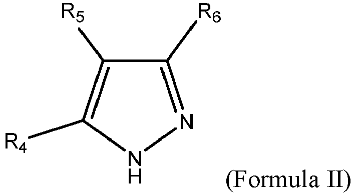 wherein each of R₄, R₅, and R₆ is independently selected from hydrogen and halogen;
wherein at least one of R₄, R₅, and R₆ is hydrogen; and
wherein the compound of Formula IV has the structure, 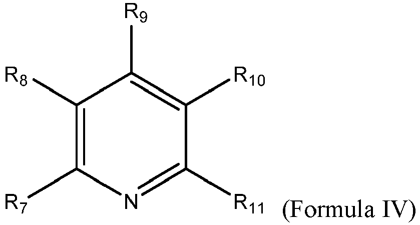 wherein each of R₇ - R₁₁ is independently selected from hydrogen and halogen; and
wherein the compound of Formula II is prepared according to a method comprising
ia) forming a mixture comprising
aa) a compound of Formula VIII, wherein 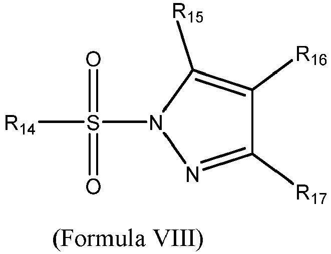
R₁₄ is selected from substituted or unsubstituted aryl, substituted or unsubstituted heteroaryl, substituted or unsubstituted carbocycle, and substituted or unsubstituted heterocycle;
each of R₁₅, R₁₆, and R₁₇ is independently selected from hydrogen and halogen; and
wherein at least one of R₁₅, R₁₆, and R₁₇ is a halogen;
bb) a solvent; and
cc) an inorganic base; and
iia) reacting the mixture;
b) a solvent;
c) an organic compound;
d) a compound comprising a metal; and
e) optionally an additive; and
ii) reacting the mixture; and
B) a metal hydroxide; and
II) reacting the mixture.

2. The method of claim 1, wherein the compound of Formula III is prepared according to a method comprising
IA) forming a mixture comprising
AA) the compound of Formula II;
BB) the compound of Formula IV;
CC) the solvent;
DD) the inorganic base; and
EE) optionally the additive; and
IIA) reacting the mixture.

3. The method of claim 1 or claim 2, wherein the metal hydroxide is selected from alkali hydroxide, alkaline earth metal hydroxide, and combinations thereof, preferably:
(A) wherein the alkali hydroxide is selected from lithium hydroxide, sodium hydroxide, and potassium hydroxide, or
(B) wherein the alkaline earth metal hydroxide is selected from calcium hydroxide and barium hydroxide.

4. The method of claim 1 or claim 2, wherein the method step II) of reacting the mixture occurs at a reaction temperature in the range of about 0 °C to about 90 °C.

5. The method of claim 1 or claim 2, wherein the solvent b) is selected from THF, toluene, 1,4-dioxane, Me-THF, and combinations thereof, preferably wherein the solvent b) is THF.

6. The method of claim 1 or claim 2, wherein the organic compound is selected from dimethyl carbonate, N,N-dimethyacetamide, and combinations thereof.

7. The method of claim 6, wherein the organic compound is dimethyl carbonate.

8. The method of claim 1 or claim 2, wherein the method step ii) of reacting the mixture occurs at a reaction temperature in the range of about 0 °C to about 60 °C, preferably wherein the method step ii) of reacting the mixture occurs at a reaction temperature in the range of about 0 °C to about 30 °C.

9. The method of claim 1 or claim 2, wherein R₅ and R₆ of Formula III are each independently hydrogen.

10. The method of claim 1 or claim 2, wherein the inorganic base DD) is selected from powder sodium hydroxide, powder potassium hydroxide, potassium carbonate, potassium phosphate, powder sodium methoxide, powder potassium *t*-butoxide, and combinations thereof.

11. A method of preparing a compound of Formula VI, wherein 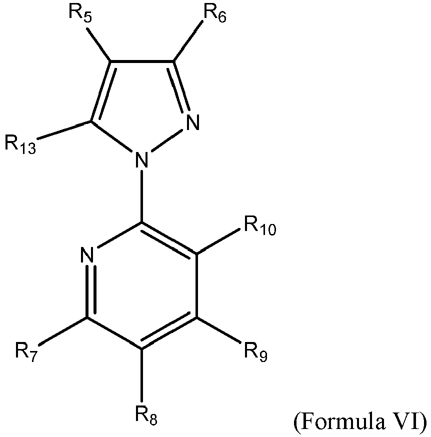 each of R₅ - R₁₀ is independently selected from hydrogen and halogen; and
R₁₃ is an organic acid, the method comprising
I) forming a mixture comprising
A) a compound of Formula III, wherein 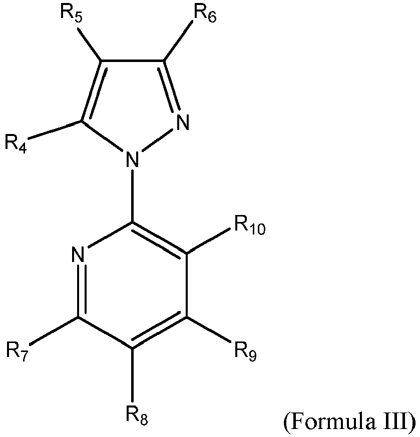
R₄ is hydrogen;
each of R₅ - R₁₀ is independently selected from hydrogen and halogen; and
wherein the compound of Formula III is prepared by mixing a compound of Formula II with a compound of Formula IV in a solvent in the presence of an inorganic base and optionally an additive,
wherein the compound of Formula II has the structure, 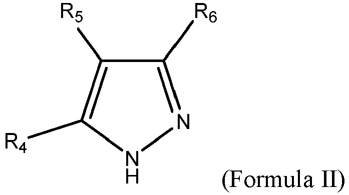 wherein each of R₄, R₅, and R₆ is independently selected from hydrogen and halogen; and
wherein at least one of R₄, R₅, and R₆ is hydrogen; and
wherein the compound of Formula IV has the structure, 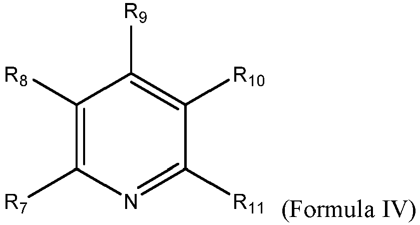 wherein each of R₇ - R₁₁ is independently selected from hydrogen and halogen; and
wherein the compound of Formula II is prepared according to a method comprising
IA) forming a mixture comprising
AA) a compound of Formula VIII, wherein 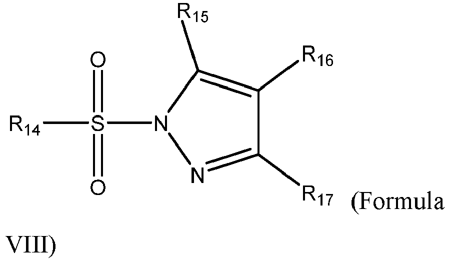
R₁₄ is selected from substituted or unsubstituted aryl, substituted or unsubstituted heteroaryl, substituted or unsubstituted carbocycle, and substituted or unsubstituted heterocycle;
each of R₁₅, R₁₆, and R₁₇ is independently selected from hydrogen and halogen; and
wherein at least one of R₁₅, R₁₆, and R₁₇ is a halogen;
BB) a solvent; and
CC) an inorganic base; and
IIa) reacting the mixture;
B) a carbonyl-containing compound;
C) a solvent;
D) a compound comprising a metal; and
E) optionally an additive; and
II) reacting the mixture.

12. The method of claim 11, wherein the compound of Formula III is prepared according to a method comprising
i) forming a mixture comprising
a) the compound of Formula II;
b) the compound of Formula IV;
c) the solvent;
d) the inorganic base; and
e) optionally the additive; and
ii) reacting the mixture.

13. The method of any of claims 1, 11 or 12, wherein the compound comprising a metal is selected from a Grignard reagent and a lithium-containing compound, preferably:
(A) wherein the Grignard reagent is selected from MeMgCl, *i*PrMgCl, *i*PrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, and combinations thereof;
more preferably wherein the Grignard reagent is *i*Pr₂NMgCl; or
(B) wherein the lithium-containing compound is selected from LDA, nBuLi, and combinations thereof.

14. A method of preparing a compound of Formula II, wherein 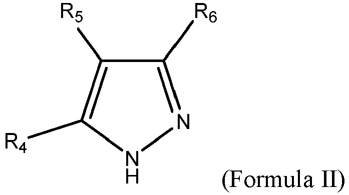 each of R₄, R₅, and R₆ is independently selected from hydrogen and halogen; and
wherein at least one of R₄, R₅, and R₆ is hydrogen, the method comprising
I) forming a mixture comprising
A) a compound of Formula VIII, wherein 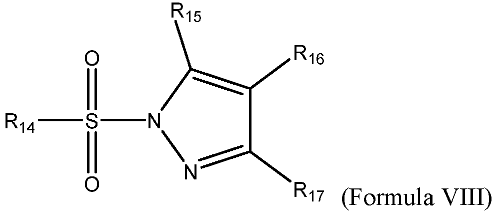
R₁₄ is selected from substituted or unsubstituted aryl, substituted or unsubstituted heteroaryl, substituted or unsubstituted carbocycle, and substituted or unsubstituted heterocycle;
each of R₁₅, R₁₆, and R₁₇ is independently selected from hydrogen and halogen; and
wherein at least one of R₁₅, R₁₆, and R₁₇ is a halogen;
B) a solvent; and
C) an inorganic base; and
II) reacting the mixture.

15. The method of claim 14, wherein the solvent is selected from THF, toluene, heptanes, Me-THF, and combinations thereof.

## Patentansprüche

1. Verfahren zur Herstellung einer Verbindung der Formel VI, wobei 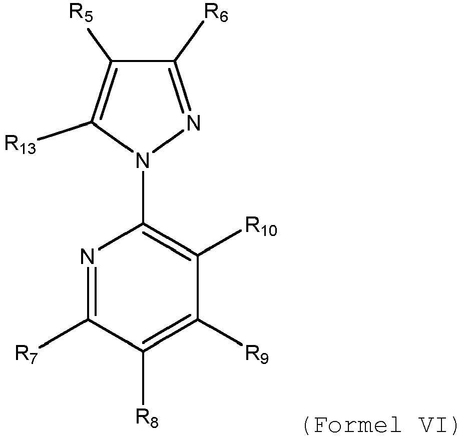 R₅ - R₁₀ jeweils unabhängig aus Wasserstoff und Halogen ausgewählt sind und
R₁₃ für eine organische Säure steht, wobei das Verfahren Folgendes umfasst:
I) Bilden einer Mischung, umfassend
A) eine Verbindung der Formel V, wobei 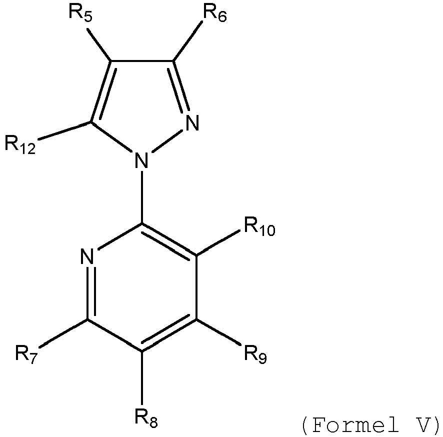 R₅ - R₁₀ jeweils unabhängig aus Wasserstoff und Halogen ausgewählt sind und
R₁₂ aus Ester und Nitril ausgewählt ist; und wobei die Verbindung der Formel V gemäß einem Verfahren hergestellt wird, das Folgendes umfasst:
i) Bilden einer Mischung, umfassend
a) eine Verbindung der Formel III, wobei 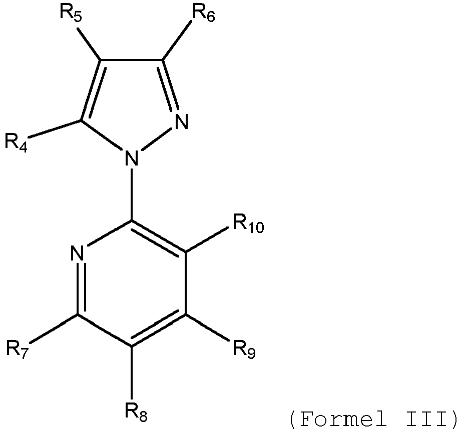 R₄ für Wasserstoff steht;
R₅ - R₁₀ jeweils unabhängig aus Wasserstoff und Halogen ausgewählt sind; und
wobei die Verbindung der Formel III durch Mischen einer Verbindung der Formel II mit einer Verbindung der Formel IV in einem Lösungsmittel in Gegenwart einer anorganischen Base und gegebenenfalls eines Additivs hergestellt wird,
wobei die Verbindung der Formel II die folgende Struktur aufweist: 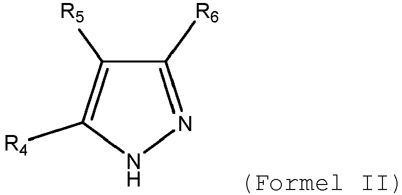 wobei R₄, R₅ und R₆ jeweils unabhängig aus Wasserstoff und Halogen ausgewählt sind und
wobei mindestens eines von R₄, R₅ und R₆ für Wasserstoff steht; und
wobei die Verbindung der Formel IV die folgende Struktur aufweist: 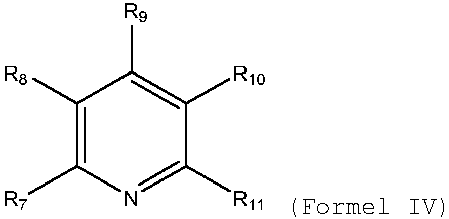 wobei R₇ - R₁₁ jeweils unabhängig aus Wasserstoff und Halogen ausgewählt sind; und
wobei die Verbindung der Formel II nach einem Verfahren hergestellt wird, das Folgendes umfasst:
ia) Bilden einer Mischung, umfassend
aa) eine Verbindung der Formel VIII, wobei 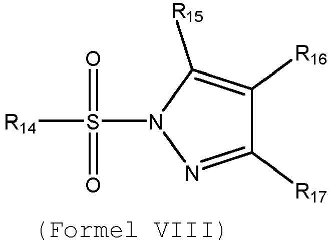
R₁₄ aus substituiertem oder unsubstituiertem Aryl, substituiertem oder unsubstituiertem Heteroaryl, substituiertem oder unsubstituiertem Carbocyclyl und substituiertem oder unsubstituiertem Heterocyclyl ausgewählt ist;
R₁₅, R₁₆ und R₁₇ jeweils unabhängig aus Wasserstoff und Halogen ausgewählt sind und
wobei mindestens eines von R₁₅, R₁₆ und R₁₇ für ein Halogen steht;
bb) ein Lösungsmittel und
cc) eine anorganische Base; und
iia) Umsetzen der Mischung;
b) ein Lösungsmittel;
c) eine organische Verbindung;
d) eine Verbindung, die ein Metall umfasst; und
e) gegebenenfalls ein Additiv; und
ii) Umsetzen der Mischung; und
B) ein Metallhydroxid; und
II) Umsetzen der Mischung.

2. Verfahren nach Anspruch 1, wobei die Verbindung der Formel III nach einem Verfahren hergestellt wird, das Folgendes umfasst:
IA) Bilden einer Mischung, umfassend
AA) die Verbindung der Formel II;
BB) die Verbindung der Formel IV;
CC) das Lösungsmittel;
DD) die anorganische Base und
EE) gegebenenfalls das Additiv; und
IIA) Umsetzen der Mischung.

3. Verfahren nach Anspruch 1 oder Anspruch 2, wobei das Metallhydroxid aus Alkalihydroxid, Erdalkalimetallhydroxid und Kombinationen davon ausgewählt wird, vorzugsweise:
(A) wobei das Alkalihydroxid aus Lithiumhydroxid, Natriumhydroxid und Kaliumhydroxid ausgewählt wird oder
(B) wobei das Erdalkalimetallhydroxid aus Calciumhydroxid und Bariumhydroxid ausgewählt wird.

4. Verfahren nach Anspruch 1 oder Anspruch 2, wobei der Verfahrensschritt II) des Umsetzens der Mischung bei einer Reaktionstemperatur im Bereich von etwa 0 °C bis etwa 90 °C erfolgt.

5. Verfahren nach Anspruch 1 oder Anspruch 2, wobei das Lösungsmittel b) aus THF, Toluol, 1,4-Dioxan, Me-THF und Kombinationen davon ausgewählt wird, vorzugsweise wobei es sich bei dem Lösungsmittel b) um THF handelt.

6. Verfahren nach Anspruch 1 oder Anspruch 2, wobei die organische Verbindung aus Dimethylcarbonat, N,N-Dimethylacetamid und Kombinationen davon ausgewählt wird.

7. Verfahren nach Anspruch 6, wobei es sich bei der organischen Verbindung um Dimethylcarbonat handelt.

8. Verfahren nach Anspruch 1 oder Anspruch 2, wobei der Verfahrensschritt ii) des Umsetzens der Mischung bei einer Reaktionstemperatur im Bereich von etwa 0 °C bis etwa 60 °C erfolgt, vorzugsweise wobei der Verfahrensschritt ii) des Umsetzens der Mischung bei einer Reaktionstemperatur im Bereich von etwa 0 °C bis etwa 30 °C erfolgt.

9. Verfahren nach Anspruch 1 oder Anspruch 2, wobei R₅ und R₆ von Formel III jeweils unabhängig für Wasserstoff stehen.

10. Verfahren nach Anspruch 1 oder Anspruch 2, wobei die anorganische Base DD) aus pulverförmigem Natriumhydroxid, pulverförmigem Kaliumhydroxid, Kaliumcarbonat, Kaliumphosphat, pulverförmigem Natriummethoxid, pulverförmigem Kalium-t-butoxid und Kombinationen davon ausgewählt wird.

11. Verfahren zur Herstellung einer Verbindung der Formel VI, wobei 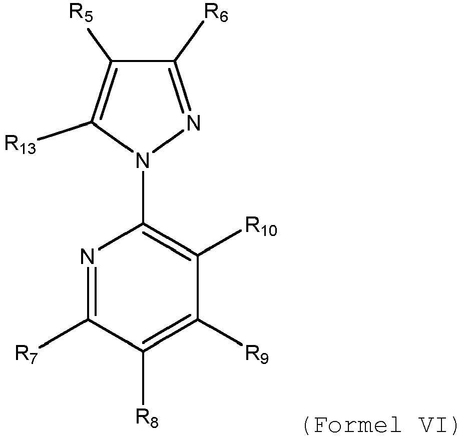 R₅ - R₁₀ jeweils unabhängig aus Wasserstoff und Halogen ausgewählt sind und
R₁₃ für eine organische Säure steht, wobei das Verfahren Folgendes umfasst:
I) Bilden einer Mischung, umfassend
A) eine Verbindung der Formel III, wobei 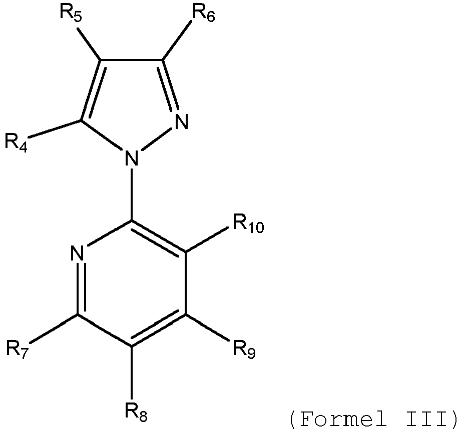
R₄ für Wasserstoff steht;
R₅ - R₁₀ jeweils unabhängig aus Wasserstoff und Halogen ausgewählt sind; und
wobei die Verbindung der Formel III durch Mischen einer Verbindung der Formel II mit einer Verbindung der Formel IV in einem Lösungsmittel in Gegenwart einer anorganischen Base und gegebenenfalls eines Additivs hergestellt wird,
wobei die Verbindung der Formel II die folgende Struktur aufweist: 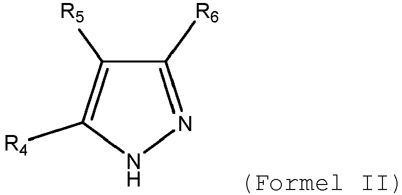 wobei R₄, R₅ und R₆ jeweils unabhängig aus Wasserstoff und Halogen ausgewählt sind und
wobei mindestens eines von R₄, R₅ und R₆ für Wasserstoff steht; und
wobei die Verbindung der Formel IV die folgende Struktur aufweist: 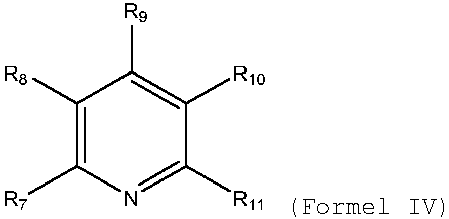 wobei R₇ - R₁₁ jeweils unabhängig aus Wasserstoff und Halogen ausgewählt sind; und
wobei die Verbindung der Formel II nach einem Verfahren hergestellt wird, das Folgendes umfasst:
IA) Bilden einer Mischung, umfassend
AA) eine Verbindung der Formel VIII, wobei 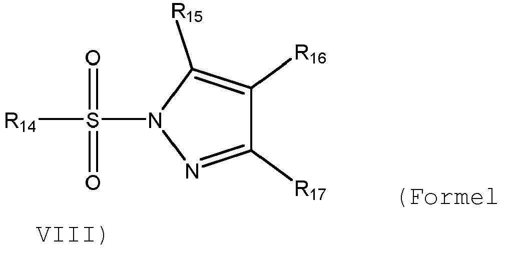
R₁₄ aus substituiertem oder unsubstituiertem Aryl, substituiertem oder unsubstituiertem Heteroaryl, substituiertem oder unsubstituiertem Carbocyclyl und substituiertem oder unsubstituiertem Heterocyclyl ausgewählt ist;
R₁₅, R₁₆ und R₁₇ jeweils unabhängig aus Wasserstoff und Halogen ausgewählt sind und
wobei mindestens eines von R₁₅, R₁₆ und R₁₇ für ein Halogen steht;
BB) ein Lösungsmittel und
CC) eine anorganische Base; und
IIa) Umsetzen der Mischung;
B) eine carbonylgruppenhaltige Verbindung;
C) ein Lösungsmittel;
D) eine Verbindung, die ein Metall umfasst; und
E) gegebenenfalls ein Additiv; und
II) Umsetzen der Mischung.

12. Verfahren nach Anspruch 11, wobei die Verbindung der Formel III nach einem Verfahren hergestellt wird, das Folgendes umfasst:
i) Bilden einer Mischung, umfassend
a) die Verbindung der Formel II;
b) die Verbindung der Formel IV;
c) das Lösungsmittel;
d) die anorganische Base und
e) gegebenenfalls das Additiv; und
ii) Umsetzen der Mischung.

13. Verfahren nach einem der Ansprüche 1, 11 oder 12, wobei die Verbindung, die ein Metall umfasst, aus einem Grignard-Reagenz und einer lithiumhaltigen Verbindung ausgewählt wird, vorzugsweise:
(A) wobei das Grignard-Reagenz aus MeMgCl, iPrMgCl, iPrMgBr, EtMgCl, iPr₂NMgCl, *i*Pr₂NMgBr, Et₂NMgCl, TMPMgCl, TMPMgCl•LiCl, *i*Pr₂NMgCl •LiCl, *i*Pr₂NMgBr•LiCl und Kombinationen davon ausgewählt wird;
weiter bevorzugt wobei es sich bei dem Grignard-Reagenz um *i*Pr₂NMgCl handelt; oder
(B) wobei die lithiumhaltige Verbindung aus LDA, nBuLi und Kombinationen davon ausgewählt wird.

14. Verfahren zur Herstellung einer Verbindung der Formel II, wobei 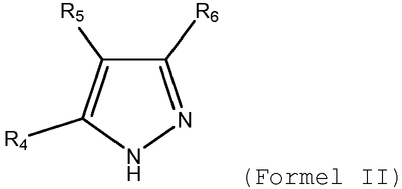 R₄, R₅ und R₆ jeweils unabhängig aus Wasserstoff und Halogen ausgewählt sind und
wobei mindestens eines von R₄, R₅ und R₆ für Wasserstoff steht, wobei das Verfahren Folgendes umfasst:
I) Bilden einer Mischung, umfassend
A) eine Verbindung der Formel VIII, wobei 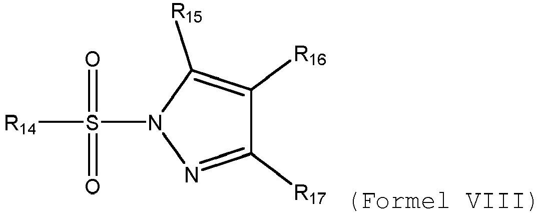 R₁₄ aus substituiertem oder unsubstituiertem Aryl, substituiertem oder unsubstituiertem Heteroaryl, substituiertem oder unsubstituiertem Carbocyclyl und substituiertem oder unsubstituiertem Heterocyclyl ausgewählt ist;
R_{15,} R₁₆ und R₁₇ jeweils unabhängig aus Wasserstoff und Halogen ausgewählt sind und wobei mindestens eines von R₁₅, R₁₆ und R₁₇ für ein Halogen steht;
B) ein Lösungsmittel und
C) eine anorganische Base; und
II) Umsetzen der Mischung.

15. Verfahren nach Anspruch 14, wobei das Lösungsmittel aus THF, Toluol, Heptangemisch, Me-THF und Kombinationen davon ausgewählt wird.

## Revendications

1. Procédé de préparation d'un composé de formule VI, 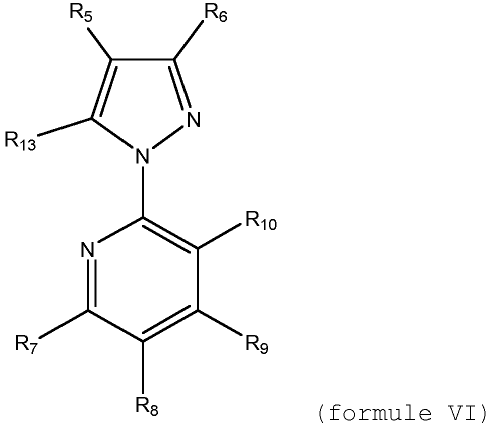 chacun parmi R₅ - R₁₀ étant indépendamment choisi parmi hydrogène et halogène ; et
R₁₃ étant un acide organique, le procédé comprenant
I) la formation d'un mélange comprenant
A) un composé de formule V, 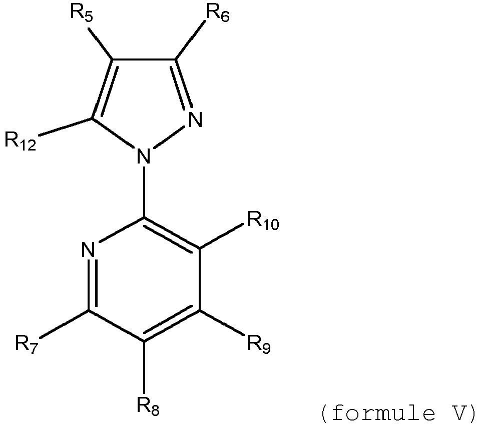
chacun parmi R₅ - R₁₀ étant indépendamment choisi parmi hydrogène et halogène ;
R₁₂ étant choisi parmi ester, et nitrile ; et
le composé de formule V étant préparé selon un procédé comprenant
i) la formation d'un mélange comprenant
a) un composé de formule III, 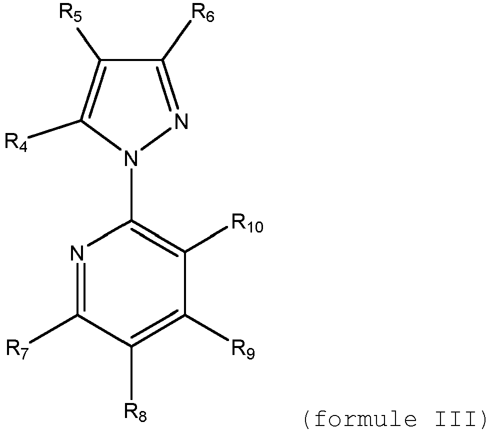 R₄ étant hydrogène ;
chacun parmi R₅ - R₁₀ étant indépendamment choisi parmi hydrogène et halogène ; et
le composé de formule III étant préparé en mélangeant un composé de formule II avec un composé de formule IV dans un solvant en la présence d'une base inorganique et éventuellement d'un additif,
le composé de formule II ayant la structure, 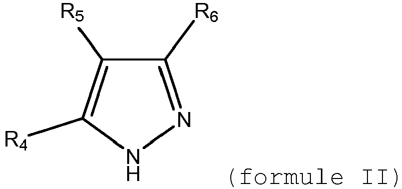
chacun parmi R₄, R₅, et R₆ étant indépendamment choisi parmi hydrogène et halogène ;
au moins l'un parmi R₄, R₅, et R₆ étant hydrogène ; et
le composé de formule IV ayant la structure, 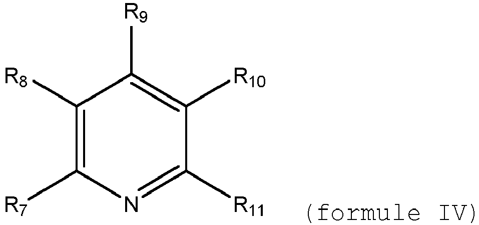 chacun parmi R₇ - R₁₁ étant indépendamment choisi parmi hydrogène et halogène ; et
le composé de formule II étant préparé selon un procédé comprenant
ia) la formation d'un mélange comprenant
aa) un composé de formule VIII, 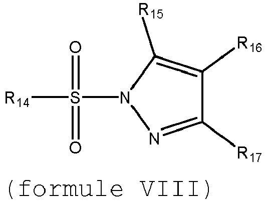
R₁₄ étant choisi parmi aryle substitué ou non substitué, hétéroaryle substitué ou non substitué, carbocycle substitué ou non substitué, et hétérocycle substitué ou non substitué ;
chacun parmi R₁₅, R₁₆, et R₁₇ étant indépendamment choisi parmi hydrogène et halogène ; et
au moins l'un parmi R₁₅, R₁₆, et R₁₇ étant un halogène ;
bb) un solvant ; et
cc) une base inorganique ; et
iia) la mise en réaction du mélange ;
b) un solvant ;
c) un composé organique ;
d) un composé comprenant un métal ; et
e) éventuellement un additif ; et
ii) la mise en réaction du mélange ; et
B) un hydroxyde métallique ; et
II) la mise en réaction du mélange.

2. Procédé selon la revendication 1, le composé de formule III étant préparé selon un procédé comprenant
IA) la formation d'un mélange comprenant
AA) le composé de formule II ;
BB) le composé de formule IV ;
CC) le solvant ;
DD) la base inorganique ; et
EE) éventuellement l'additif ; et
IIA) la mise en réaction du mélange.

3. Procédé selon la revendication 1 ou la revendication 2, l'hydroxyde métallique étant choisi parmi un hydroxyde d'alcali, un hydroxyde de métal alcalino-terreux, et des combinaisons correspondantes, préférablement :
(A) l'hydroxyde d'alcali étant choisi parmi l'hydroxyde de lithium, l'hydroxyde de sodium et l'hydroxyde de potassium, ou
(B) l'hydroxyde de métal alcalino-terreux étant choisi parmi l'hydroxyde de calcium et l'hydroxyde de baryum.

4. Procédé selon la revendication 1 ou la revendication 2, l'étape du procédé II) de mise en réaction du mélange ayant lieu à une température de réaction dans la plage d'environ 0 °C à environ 90 °C.

5. Procédé selon la revendication 1 ou la revendication 2, le solvant b) étant choisi parmi le THF, le toluène, le 1,4-dioxane, le Me-THF, et des combinaisons correspondantes, préférablement le solvant b) étant le THF.

6. Procédé selon la revendication 1 ou la revendication 2, le composé organique étant choisi parmi le carbonate de diméthyle, le N,N-diméthylacétamide, et des combinaisons correspondantes.

7. Procédé selon la revendication 6, le composé organique étant le carbonate de diméthyle.

8. Procédé selon la revendication 1 ou la revendication 2, l'étape du procédé ii) de mise en réaction du mélange ayant lieu à une température de réaction dans la plage d'environ 0 °C à environ 60 °C, préférablement l'étape du procédé ii) de mise en réaction du mélange ayant lieu à une température de réaction dans la plage d'environ 0 °C à environ 30 °C.

9. Procédé selon la revendication 1 ou la revendication 2, R₅ et R₆ de la formule III étant chacun indépendamment hydrogène.

10. Procédé selon la revendication 1 ou la revendication 2, la base inorganique DD) étant choisie parmi l'hydroxyde de sodium en poudre, l'hydroxyde de potassium en poudre, le carbonate de potassium, le phosphate de potassium, le méthoxyde de sodium en poudre, le t-butoxyde de potassium en poudre, et des combinaisons correspondantes.

11. Procédé de préparation d'un composé de formule VI, 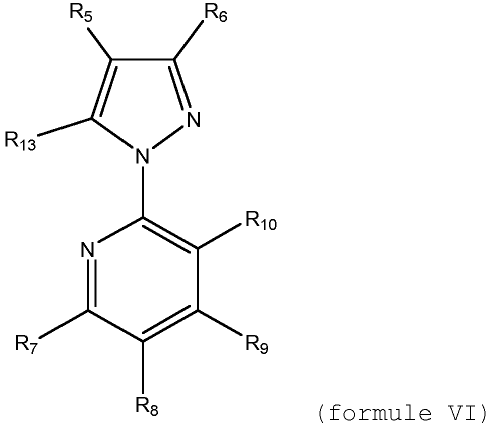 chacun parmi R₅ - R₁₀ étant indépendamment choisi parmi hydrogène et halogène ; et
R₁₃ étant un acide organique, le procédé comprenant
I) la formation d'un mélange comprenant
A) un composé de formule III, 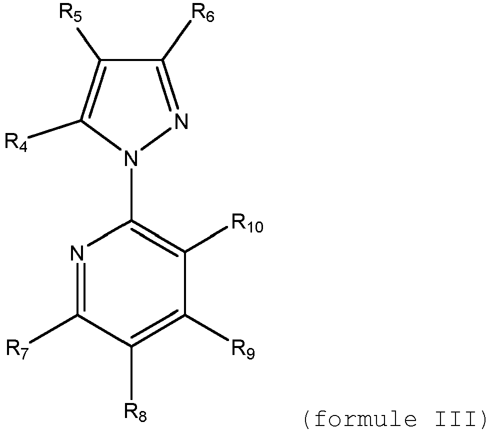
R₄ étant hydrogène ;
chacun parmi R₅ - R₁₀ étant indépendamment choisi parmi hydrogène et halogène ; et
le composé de formule III étant préparé en mélangeant un composé de formule II avec un composé de formule IV dans un solvant en la présence d'une base inorganique et éventuellement d'un additif,
le composé de formule II ayant la structure, 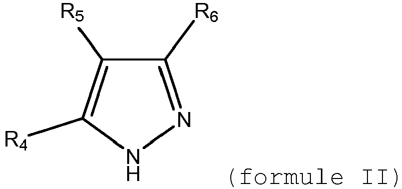 chacun parmi R₄, R₅, et R₆ étant indépendamment choisi parmi hydrogène et halogène ; et
au moins l'un parmi R₄, R₅, et R₆ étant hydrogène ; et
le composé de formule IV ayant la structure, 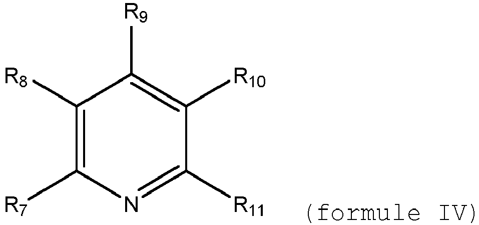 chacun parmi R₇ - R₁₁ étant indépendamment choisi parmi hydrogène et halogène ; et le composé de formule II étant préparé selon un procédé comprenant
IA) la formation d'un mélange comprenant
AA) un composé de formule VIII, 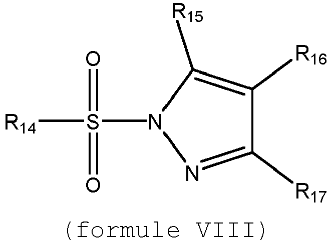
R₁₄ étant choisi parmi aryle substitué ou non substitué, hétéroaryle substitué ou non substitué, carbocycle substitué ou non substitué, et hétérocycle substitué ou non substitué ;
chacun parmi R₁₅, R₁₆, et R₁₇ étant indépendamment choisi parmi hydrogène et halogène ; et
au moins l'un parmi R₁₅, R₁₆, et R₁₇ étant un halogène ;
BB) un solvant ; et
CC) une base inorganique ; et
IIa) la mise en réaction du mélange ;
B) un composé contenant un carbonyle ;
C) un solvant ;
D) un composé comprenant un métal ; et
E) éventuellement un additif ; et
II) la mise en réaction du mélange.

12. Procédé selon la revendication 11, le composé de formule III étant préparé selon le procédé comprenant
i) la formation d'un mélange comprenant
a) le composé de formule II ;
b) le composé de formule IV ;
c) le solvant ;
d) la base inorganique ; et
e) éventuellement l'additif ; et
ii) la mise en réaction du mélange.

13. Procédé selon l'une quelconque des revendications 1, 11 ou 12, le composé comprenant un métal étant choisi parmi un réactif de Grignard et un composé contenant du lithium, préférablement :
(A) le réactif de Grignard étant choisi parmi MeMgCl, *i*PrMgCl, *i*PrMgBr, EtMgCl, iPr₂NMgCl, *i*Pr₂NMgBr, Et₂NMgCl, TMPMgCl, TMPMgCl•LiCl, *i*Pr₂NMgCl•LiCl, *i*Pr₂NMgBr•LiCl, et des combinaisons correspondantes ;
plus préférablement le réactif de Grignard étant *i*Pr₂NMgCl ; ou
(B) le composé contenant du lithium étant choisi parmi LDA, nBuLi, et des combinaisons correspondantes.

14. Procédé de préparation d'un composé de formule II, 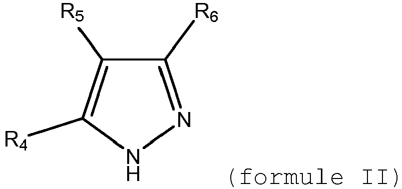 chacun parmi R₄, R₅, et R₆ étant indépendamment choisi parmi hydrogène et halogène ; et
au moins l'un parmi R₄, R₅, et R₆ étant hydrogène, le procédé comprenant
I) la formation d'un mélange comprenant
A) un composé de formule VIII, 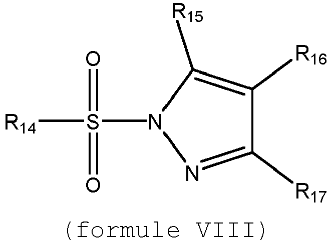
R₁₄ étant choisi parmi aryle substitué ou non substitué, hétéroaryle substitué ou non substitué, carbocycle substitué ou non substitué, et hétérocycle substitué ou non substitué ;
chacun parmi R₁₅, R₁₆, et R₁₇ étant indépendamment choisi parmi hydrogène et halogène ; et
au moins l'un parmi R₁₅, R₁₆, et R₁₇ étant un halogène ;
B) un solvant ; et
C) une base inorganique ; et
II) la mise en réaction du mélange.

15. Procédé selon la revendication 14, le solvent étant choisi parmi le THF, le toluène, les heptanes, le Me-THF, et des combinaisons correspondantes.
